# EUROPEAN PATENT APPLICATION

(11) **EP 3 473 245 A1**
(43) Date of publication of application: **24.04.2019**
(21) Application number: 18201506.5
(22) Date of filing: 19.10.2018
(51) Int. Cl.: A61K 9/16, A61K 31/18, A61P 13/08

(54) **CONTROLLED RELEASE ORAL TAMSULOSIN HYDROCHLORIDE**

(30) Priority: 20.10.2017 EP 17197508; 08.11.2017 EP 17200481
(71) Applicant: Veru Inc., Miami, Florida 33137 (US)
(72) Inventor: BARNETTE, Gary K., Wake Forest, NC 27587 (US); STEVENS, Ruth E., Cincinnati OH 45242 (US); PHELPS, Kenneth V., Cincinnati OH (US); GOLD, Lynn, Seattle WA 98103 (US)
(74) Representative: Kutzenberger Wolff & Partner

(57) **Abstract**

The present invention relates in certain embodiments to a controlled release formulation, especially a sachet, comprising a unit dosage of a dry powder of Tamsulosin or a pharmaceutically acceptable salt thereof in a controlled release matrix and to methods of making and using such formulation. The controlled release formulation is beneficial in the treatment of benign prostatic hyperplasia (BPH), particularly in those patients suffering from dysphagia. The controlled release formulation is easily dispersed in water or other suitable liquid, and so solves the problem of dysphagia, thereby improving patient compliance in that targeted patient population, yet the controlled release formulation has a release profile in a patient on an empty stomach similar to FLOMAX® ((R)-5-(2-{[2-(2-Ethoxyphenoxy)ethyl]amino}propyl)-2-methoxybenzene-1-sulfonamide hydrochloride) taken 30 minutes after a meal, but does not exhibit the FLOMAX® tablet food effect, thereby improving dosage form administration flexibility.

## Description

The present invention relates in one embodiment to formulations of Tamsulosin hydrochloride, and, more specifically, to oral controlled release formulations of Tamsulosin hydrochloride, and, especially, to oral controlled release suspension formulations of Tamsulosin hydrochloride for patients that suffer from dysphagia.

Tamsulosin is an alphal adrenoreceptor antagonist that is approved by the FDA for the treatment of signs and symptoms of benign prostatic hyperplasia (BPH). The approved product, which is marketed in the United States as FLOMAX® ((R)-5-(2-{[2-(2-Ethoxyphenoxy)ethyl]amino}propyl)-2-methoxybenzene-1-sulfonamide hydrochloride), is an oral capsule (extended release) containing 0.4 mg of Tamsulosin hydrochloride. The dosage and administration section of the approved prescribing information states that the capsules should not be crushed, chewed or opened.

Benign prostatic hyperplasia (BPH) is a condition where the stromal and epithelial cells in the prostate are enlarged. As the hyperplasia worsens, the urethra is impinged resulting in increased resistance to the flow of urine through the urethra. This results in incomplete voiding of urine, difficulty in urination, and may lead to atrophy of the muscle in the bladder wall, and to instability and weakness of the bladder wall. BPH is a common condition in men with the incidence and prevalence increasing with age.

There is a subset of the population that cannot swallow pills (capsules and tablets). The inability to swallow or difficulty swallowing is called dysphagia. Dysphagia may be a result of choking or fear of choking or may be due to comorbidities such as dry throat, blockage in the mouth or throat, and a number of other conditions and diseases. The comorbidities leading to dysphagia cause the incidence of dysphagia in the elderly to be increased, but the condition is not limited to the elderly and may afflict people of any age.

Currently, Tamsulosin is only available in capsule form (extended release) and there are express instructions not to crush, chew, or open the capsule in the prescribing information of the approved products. Therefore, people that suffer from dysphagia that require Tamsulosin for the treatment of signs and symptoms of benign prostatic hyperplasia will find no treatment or formulation option that will allow them to take Tamsulosin.

Tamsulosin was first described in U.S. Patent No. 4,703,063.

Controlled release Tamsulosin formulations are also described in the prior art. For example, U.S. Patent No. 4,772,475 describes a controlled released formulation of Tamsulosin, microcrystalline cellulose and a release controlling agent, for example, a (meth)acrylic acid copolymer, such as EUDRAGIT® (methacrylate copolymer), granulated and formed into tablets and capsules. U.S. Patent No. 7,018,658 describes a similar product, the distinction being the granules of the '658 patent comprise a core of Tamsulosin that is coated with a shell of the release controlling agent. According to the '658 patent, the release profile of the dosage form of the '475 patent is not sufficient for an extended release dosage form (see the '658 patent at col. 2, 11 2-12), and a pellet inner core surrounded by an outer shell of a release controlling agent provides effective extended release (see the '658 patent at col. 3, 11 5-25.) Fleeting mention is made to "sachets" (see the '658 patent at col. 6,11 14-16), but no details are provided how the sachets might be constructed, or how the granules might be administered in this form. Thus, capsules are themselves sometimes packaged in single-dosage sachets and this may be what is meant in view of the later discussion in the '658 patent at col. 6,1130-39, of a suitable package comprising a unit dosage amount of Tamsulosin, including blister packs and plastic or glass bottles. There is no indication at any point that the Tamsulosin is administered in the form of a liquid or that such liquid is the result of suspending a Tamsulosin powder in a precursor liquid. Indeed, all examples relate to coated pellets.

WO 2004/056354 relates to controlled release pharmaceutical compositions of Tamsulosin or its pharmaceutically acceptable salts thereof, more particularly to a controlled release individual unit or multiple unit formulation comprising a spherical core obtained by adding release controlling agent to a mixture of Tamsulosin and spheronizing agent.

WO 2006/005512 relates to granules for controlled release of Tamsulosin comprising Tamsulosin and a carrier matrix. The carrier matrix comprises 2 to 25% by weight of an alginate, 30 to 70% by weight of a macromolecular substance and 10 to 50% by weight of a hydrophobic substance. The macromolecular substance is selected from the group of methacrylic acid/ethyl acrylate 1:1 copolymer, methacrylic acid/methyl methacrylate 1:1 or 1:2 copolymers, aminoalkyl methacrylate copolymer, vinyl acetate/crotonic acid copolymer, polyvinyl acetate phthalate, ethylene-vinyl acetate, cellulose acetate phthalate, hydroxypropylmethylcellulose, sodium carboxymethylcellulose, carrageenan, crosslinked chitosan, polyethylene-vinyl acetate, poly-L-lactic acid, xanthan gum and polyvinyl acetate or mixtures thereof. The hydrophobic substance is selected from the group of glycerol behenate, glyceryl monostearate, wax, mono-, di- and trisubstituted glycerides and calcium stearate or mixtures thereof.

WO 2013/123965 relates to a dosage form comprising a fixed dose combination of a 5-alpha reductase inhibitor and an alpha-adrenergic blocker components, more in particular dutasteride and Tamsulosin, said dosage form comprising an inner hard-shell capsule being placed in an outer hard-shell capsule, wherein one component is placed in the inner capsule and the second component is placed in the space between the inner and outer hard-shell capsules.

EP 1 568 361 discloses a sustained-release pharmaceutical composition containing Tamsulosin or a pharmaceutically acceptable salt thereof and a carrier for a sustained-release pharmaceutical composition and, when dissolution test is carried out according to Japanese Pharmacopoeia Dissolution Test Method 2, the Tamsulosin release after 7 hours from the start of the dissolution is about 20 to about 85%.

EP 2 837 379 describes dosage forms containing a steroid 5-alpha-reductase inhibitor, in combination with an alpha blocker, for the treatment or prophylaxis of an androgen mediated disease or condition, preferably for the treatment of benign prostatic hyperplasia (HPB).

US 2005/0186275 relates to a sustained release Tamsulosin formulation containing Tamsulosin, a hydrophobic polymer, a microsphere forming agent and a diluent. The hydrophobic polymers include pH-dependent and pH-independent polymers used as the release-modulating agent to control the dissolution profile of Tamsulosin formulation so that the formulation releases Tamsulosin slowly and continuously as the formulation passed through the stomach and gastrointestinal tract.

A suspension formulation of Tamsulosin that is not a tablet or capsule and can be more easily swallowed will provide patients that suffer from dysphagia with a formulation option that will allow them to take Tamsulosin and receive the medication that they need.

FLOMAX® also exhibits distinct pH-dependent drug release. Administration to humans before food intake results in a 30% increase of bioavailability and a 40-70% increase in Cmax as compared to after food intake (Physicians' Desk Reference 2002). As a result, labeling on FLOMAX® directs the tablets be taken approximately one-half hour following the same meal each day.

A formulation of Tamsulosin that is free of these mealtime ("food effect") constraints should increase patient compliance and afford administration flexibility.

Tamsulosin hydrochloride is also available in some countries as HARNAL® OCAS ((R)-5-(2-{[2-(2-Ethoxyphenoxy)ethyl]amino}propyl)-2-methoxybenzene-1-sulfonamide hydrochloride oral controlled absorption system). HARNAL® OCAS is a film-coated, prolonged release tablet containing macrogol 7,000,000, macrogol 8,000, magnesium stearate, butylated hydroxytoluene, colloidal silica anhydrous, hypromellose, and iron oxide yellow. HARNAL® OCAS tablets are purported to be only slightly affected by food and in a manner that is unlikely to be clinically significant.

HARNAL® OCAS tablets present the same challenges as FLOMAX® tablets for men suffering dysphagia.

There is a demand for pharmaceutical dosage forms of Tamsulosin or a physiologically acceptable salt thereof that have advantages compared to the pharmaceutical dosage forms of the prior art. The pharmaceutical dosage forms should be safe, reliable and therapeutically effective, provide controlled release of Tamsulosin or the physiologically acceptable salt thereof over an extended period of time, be inexpensive and easy to manufacture, require a low number of excipients, and provide excellent patient compliance. Further, the pharmaceutical dosage forms should be bioequivalent to FLOMAX® but unlike FLOMAX®, not have a pronounced food effect.

It is an object of the invention to provide improved pharmaceutical dosage forms of Tamsulosin or a physiologically acceptable salt thereof.

This object has been achieved by the subject-matter of the patent claims.

It has been surprisingly found that pharmaceutical dosage forms of Tamsulosin or a physiologically acceptable salt thereof can be manufactured that have advantages compared to the pharmaceutical dosage forms of the prior art. The pharmaceutical dosage forms according to the invention are safe, reliable and therapeutically effective, provide controlled release of Tamsulosin or the physiologically acceptable salt thereof over an extended period of time, are inexpensive and easy to manufacture, require a low number of excipients, and provide excellent patient compliance. Further, the pharmaceutical dosage forms according to the invention are bioequivalent to FLOMAX® but unlike FLOMAX®, do not have a pronounced food effect.

A first aspect of the invention relates to a pharmaceutical dosage form comprising a plurality of particles providing controlled release of Tamsulosin or a physiologically acceptable salt thereof; wherein each of said particles comprises a controlled release matrix in which the Tamsulosin or the physiologically acceptable salt thereof is embedded; wherein said controlled release matrix comprises a mixture of a first cellulose derivative and a second cellulose derivative; wherein said first cellulose derivative differs from said second cellulose derivative; wherein said first cellulose derivative and said second cellulose derivative each are independently of one another selected from the group consisting of cellulose ethers and cellulose esters; and wherein the total content of said first cellulose derivative and said second cellulose derivative is at least 50 wt.-%, relative to the total weight of the plurality of particles; and wherein the particles are not coated with an enteric coating material.

In another embodiment, said controlled release matrix comprises a mixture of a first cellulose and a second cellulose, wherein said first cellulose differs from said second cellulose; and wherein the total content of said first cellulose and said second cellulose is at least 50 wt.-%, relative to the total weight of the plurality of particles; and wherein the particles are not coated with an enteric coating material. The first cellulose and the second cellulose can be selected from a group including, for example, but not limited to a microcrystalline cellulose (MCC), powdered cellulose (PC), and low crystallinity powdered cellulose (LCPC). Examples of a microcrystalline cellulose include, but not limited to, silisified MCC (SMCC) and MCC type II (MCC-II).

The pharmaceutical dosage form according to the invention comprises a plurality of particles, depending upon the individual particle size and weight, typically dozens, hundreds or thousands of particles.

Preferably, all particles within said plurality of particles have essentially the same chemical composition, i.e. preferably contain the same form of Tamsulosin and the same excipients in the same relative amounts.

Each of said particles within said plurality of particles comprises a controlled release matrix in which the Tamsulosin or the physiologically acceptable salt thereof is embedded. For the purpose of the specification, "*controlled release*" means "*prolonged release*" and/or "*delayed release*"*.* Specifically, "*controlled release*" means that under *in vitro* conditions, preferably in accordance with Ph. Eur. in 500 mL buffer at 100 rpm and at 37 °C, for the first 2 hours in buffer containing 0.003% polysorbate 80 at pH 1.2 and for the subsequent 6 hours in phosphate buffer at pH 7.2, more preferably as specified in Example 7,
- after 6 hours have released from 40 wt.-% to 100 wt.-%, more preferably from 50 wt.-% to 90 wt.-%, still more preferably from 65 wt.-% to 85 wt.-% of the total amount of the Tamsulosin or the physiologically acceptable salt thereof that was originally contained in the pharmaceutical dosage form; and
- after 8 hours have released not less than 70 wt.-%, more preferably not less than 80 wt.-% of the total amount of the Tamsulosin or the physiologically acceptable salt thereof that was originally contained in the pharmaceutical dosage form.

In one embodiment, the pharmaceutical dosage form according to the invention under *in vivo* conditions provides the average pharmacokinetic parameters Cmax, Tmax and AUCτ, the values of which are similar to one another under fed status and under fasted status, such that there is no pronounced food effect, wherein Cmax = observed maximum Tamsulosin hydrochloride plasma concentration, wherein Tmax = median time-to-maximum concentration, and wherein AUCτ = Area under the Tamsulosin hydrochloride plasma time curve over the dosing interval. Preferably, under fed status as well as under fasted status, the following parameters are provided:
a) Tamsulosin HCl at a dose of 0.4 mg:
   - Cmax: 10±8 ng/ml; more preferably 10±6 ng/ml; most preferably 10±4 ng/ml;
   - Tmax: 6.0±3 h; more preferably 6.0±2 h; most preferably 6.0±1 h;
   - AUCτ: 150±120 ng•hr/mL; more preferably 150±80 ng•hr/mL; most preferably 150±40 ng•hr/mL.
b) Tamsulosin HCl at a dose of 0.8 mg:
   - Cmax: 30±24 ng/ml; more preferably 30±18 ng/ml; most preferably 30±12 ng/ml;
   - Tmax: 7.0±3 h; more preferably 7.0±2 h; most preferably 7.0±1 h;
   - AUCτ: 450±360 ng•hr/mL; more preferably 450±240 ng•hr/mL; most preferably 450±120 ng•hr/mL.

The controlled release matrix of the particles according to the invention comprises a mixture of a first cellulose derivative and a second cellulose derivative; wherein said first cellulose derivative differs from said second cellulose derivative. For the purpose of the specification, a cellulose derivative differs from cellulose and preferably is linked to one or more different moieties through ether and/or ester bonds. Thus, a cellulose derivative is a chemically derivatized cellulose. The first cellulose derivative and the second cellulose derivative each are independently of one another selected from the group consisting of cellulose ethers and cellulose esters.

Preferably, said first cellulose derivative and said second cellulose derivative each are independently of one another selected from the group consisting of ethyl cellulose, cellulose acetate phthalate, hydroxypropyl methyl cellulose, hydroxypropyl methyl cellulose acetate succinate, and hydroxypropyl methyl cellulose phthalate, carboxymethyl cellulose, carboxyethyl cellulose, butyl cellulose, ethyl cellulose acetate, ethyl cellulose propionate, methyl cellulose propionate, cellulose acetate, cellulose diacetate, cellulose triacetate, cellulose acetate-propionate, cellulose butyrate, cellulose isobutyrate, and cellulose acetate-butyrate. Numerous additional cellulose derivatives are known in the art and described, for example, in U.S. Patents Nos. 3,298,979, 7,259,257, 8,617,851, and 9,469,693, and U.S. Patent Application Publications 2004/0058420, and 2005/0022952, all of which are incorporated by reference herein in their entirety.

In one embodiment,
- the first cellulose derivative is ethyl cellulose; and/or
- the second cellulose derivative is selected from the group consisting of hydroxypropyl methyl cellulose, hydroxypropyl methyl cellulose acetate succinate, and hydroxypropyl methyl cellulose phthalate.

In a particular embodiment,
- the first cellulose derivative is ethyl cellulose; and
- the second cellulose derivative is hydroxypropyl methyl cellulose acetate succinate.

In one aspect, the first cellulose derivative and the second cellulose derivative are the only cellulose derivatives that are contained in the controlled release matrix, i.e. the latter preferably comprises no additional cellulose derivatives besides said first cellulose derivative and said second cellulose derivative.

In one embodiment, said plurality of particles that are contained in the pharmaceutical dosage form according to the invention comprises said first cellulose derivative, said second cellulose derivative and Tamsulosin or the physiologically acceptable salt thereof.

In another embodiment, said plurality of particles that are contained in the pharmaceutical dosage form according to the invention comprises said first cellulose, said second cellulose and Tamsulosin or the physiologically acceptable salt thereof.

The total content of said first cellulose or its derivative and said second cellulose or its derivative, i.e. the sum of the content of said first cellulose or its derivative and of the content of said second cellulose or its derivative, is at least 50 wt.-%, relative to the total weight of the plurality of particles. Preferably, the total content of said first cellulose or its derivative and said second cellulose or its derivative is at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%, in each case relative to the total weight of the plurality of particles.

In one embodiment , the relative weight ratio of said first cellulose or its derivative to said second cellulose or its derivative is within the range of from 5:1 to 1:5; preferably from 4.5:1 to 1:4.5; more preferably from 4:1 to 1:4, still more preferably from 3.5:1 to 1:3.5, yet more preferably from 3:1 to 1:3, even more preferably from 2.5:1 to 1:2.5, most preferably from 2:1 to 1:2, and in particular from 1.5:1 to 1:1.5.

In some embodiments of the pharmaceutical dosage form according to the invention
- the content of said first cellulose or its derivative is at least 10 wt.-%, more preferably at least 15 wt.-%, still more preferably at least 20 wt.-%, yet more preferably at least 25 wt.-%, even more preferably at least 30 wt.-%, most preferably at least 35 wt.-%, and in particular at least 40 wt.-%, relative to the total weight of the plurality of particles; and/or
- the content of said second cellulose or its derivative is at least 10 wt.-%, more preferably at least 15 wt.-%, still more preferably at least 20 wt.-%, yet more preferably at least 25 wt.-%, even more preferably at least 30 wt.-%, most preferably at least 35 wt.-%, and in particular at least 40 wt.-%, relative to the total weight of the plurality of particles; and/or
- the content of Tamsulosin or the physiologically acceptable salt thereof is at least 3.0 wt.-%, more preferably at least 4.0 wt.-%, still more preferably at least 5.0 wt.-%, yet more preferably at least 6.0 wt.-%, even more preferably at least 7.0 wt.-%, most preferably at least 8.0 wt.-%, and in particular at least 9.0 wt.-%, relative to the total weight of the plurality of particles.

In some embodiments of the pharmaceutical dosage form according to the invention,
- the content of said first cellulose or its derivative is within the range of 45±40 wt.-%, preferably 45±35 wt.-%, more preferably 45±30 wt.-%, still more preferably 45±25 wt.-%, yet more preferably 45±20 wt.-%, even more preferably 45±15 wt.-%, most preferably 45±10 wt.-%, and in particular 45±5 wt.-%, relative to the total weight of the plurality of particles; and/or
- the content of said second cellulose or its derivative is within the range of 45±40 wt.-%, preferably 45±35 wt.-%, more preferably 45±30 wt.-%, still more preferably 45±25 wt.-%, yet more preferably 45±20 wt.-%, even more preferably 45±15 wt.-%, most preferably 45±10 wt.-%, and in particular 45±5 wt.-%, relative to the total weight of the plurality of particles; and/or
- the content of the Tamsulosin or the physiologically acceptable salt thereof is within the range of 10±8 wt.-%, preferably 10±7 wt.-%, more preferably 10±6 wt.-%, still more preferably 10±5 wt.-%, yet more preferably 10±4 wt.-%, even more preferably 10±3 wt.-%, most preferably 10±2 wt.-%, and in particular 10±1 wt.-%, relative to the total weight of the plurality of particles, relative to the total weight of the plurality of particles.

In particular embodiments A¹ to A⁸, the plurality of particles comprises, preferably essentially consists of, more preferably consists of the following constituents at the following contents, wherein all contents are provided in wt.-%, relative to the total weight of the plurality of particles:

| [wt.-] | A¹ | A² | A³ | A⁴ | A⁵ | A⁶ | A⁷ | A⁸ |
|---|---|---|---|---|---|---|---|---|
| first cellulose derivative | 45±40 | 45±35 | 45±30 | 45±25 | 45±20 | 45±15 | 45±10 | 45±5 |
| second cellulose derivative | 45±40 | 45±35 | 45±30 | 45±25 | 45±20 | 45±15 | 45±10 | 45±5 |
| Tamsulosin or physiologically acceptable salt thereof | 10±8 | 10±7 | 10±6 | 10±5 | 10±4 | 10±3 | 10±2 | 10±1 |

In particular embodiments B¹ to B⁸, the first cellulose derivative is ethyl cellulose and the second cellulose derivative is selected from the group consisting of hydroxypropyl methyl cellulose, hydroxypropyl methyl cellulose acetate succinate, and hydroxypropyl methyl cellulose phthalate, wherein the plurality of particles comprises, preferably essentially consists of, more preferably consists of the following constituents at the following contents, wherein all contents are provided in wt.-%, relative to the total weight of the plurality of particles:

| [wt.-] | B¹ | B² | B³ | B⁴ | B⁵ | B⁶ | B⁷ | B⁸ |
|---|---|---|---|---|---|---|---|---|
| ethyl cellulose | 45±40 | 45±35 | 45±30 | 45±25 | 45±20 | 45±15 | 45±10 | 45±5 |
| second cellulose derivative | 45±40 | 45±35 | 45±30 | 45±25 | 45±20 | 45±15 | 45±10 | 45±5 |
| Tamsulosin or physiologically acceptable salt thereof | 10±8 | 10±7 | 10±6 | 10±5 | 10±4 | 10±3 | 10±2 | 10±1 |

In particular embodiments C¹ to C⁸, the first cellulose derivative is a cellulose ether and the second cellulose derivative is hydroxypropyl methyl cellulose acetate succinate, wherein the plurality of particles comprises, preferably essentially consists of, more preferably consists of the following constituents at the following contents, wherein all contents are provided in wt.-%, relative to the total weight of the plurality of particles:

| [wt.-] | C¹ | C² | C³ | C⁴ | C⁵ | C⁶ | C⁷ | C⁸ |
|---|---|---|---|---|---|---|---|---|
| first cellulose derivative | 45±40 | 45±35 | 45±30 | 45±25 | 45±20 | 45±15 | 45±10 | 45±5 |
| hydroxypropyl methyl cellulose acetate succinate | 45±40 | 45±35 | 45±30 | 45±25 | 45±20 | 45±15 | 45±10 | 45±5 |
| Tamsulosin or physiologically acceptable salt thereof | 10±8 | 10±7 | 10±6 | 10±5 | 10±4 | 10±3 | 10±2 | 10±1 |

In particular embodiments D¹ to D⁸, the plurality of particles comprises, preferably essentially consists of, more preferably consists of the following constituents at the following contents, wherein all contents are provided in wt.-%, relative to the total weight of the plurality of particles:

| [wt.-] | D¹ | D² | D³ | D⁴ | D³ | D⁶ | D⁷ | D⁸ |
|---|---|---|---|---|---|---|---|---|
| ethyl cellulose | 45±40 | 45±35 | 45±30 | 45±25 | 45±20 | 45±15 | 45±10 | 45±5 |
| hydroxypropyl methyl cellulose acetate succinate | 45±40 | 45±35 | 45±30 | 45±25 | 45±20 | 45±15 | 45±10 | 45±5 |
| Tamsulosin or physiologically acceptable salt thereof | 10±8 | 10±7 | 10±6 | 10±5 | 10±4 | 10±3 | 10±2 | 10±1 |

In one embodiment, said plurality of particles that are contained in the pharmaceutical dosage form according to the invention are not coated with any coating material.

In another embodiment, said plurality of particles that are contained in the pharmaceutical dosage form according to the invention do not contain one or more materials selected from the group consisting of microcrystalline cellulose, polyethylene oxide, alginic acid, alginate salts, lactose, and glycerol behenate; preferably do not contain any of the foregoing materials.

In one embodiment, said plurality of particles are free flowing, non-compacted particles.

In one embodiment, said plurality of particles have a particle size in the range of from 100 µm to 600 µm, preferably 150 µm to 450 µm. As used herein, the term "particle size" may refer to a mean particle diameter.

In one embodiment, said plurality of particles that are contained in the pharmaceutical dosage form according to the invention have an average particle size within the range of from 160 µm to 415 µm, preferably from 175 µm to 400 µm. Preferred average particle sizes of the particles are 150±50 µm, 175±50 µm, 200±50 µm, 225±50 µm, 250±50 µm, 275±50 µm, 300±50 µm, 325±50 µm, 350±50 µm, 375±50 µm, or 400±50 µm. Suitable methods to measure the average particle size of a powder are known to the skilled artisan and include laser diffraction, image analysis, mechanical sieving, optical microscopy, ultracentrifugation, sedimentation, air permeability, electron microscopy, scanning electron microscopy, and Coulter Counter techniques. For a general review of methods for determining particle size, see Martin et al., Physical Pharmacy, 3rd Ed., Lea & Febiger, Philadelphia (1983), *see also* O'Conner et al. Chpt. 88, Remington's Pharmaceutical Sciences, 18th Edition, Mack Publishing Co., Easton, PA (1990). Preferably, for the purpose of the specification, the "average particle size" is expressed in terms of the geometric mean value D[4,3] with the volume as the basis for the distribution calculation (volume distribution) in accordance with, for example, ASTM E 799. Measurement of the average particle size can also be made according to the International Standard ISO 13320:2009. For example, Malvern Instruments' laser diffraction systems can advantageously be used. Suitable Malvern systems include the Malvern 2000, MalvernMasterSizer (such as Mastersizer S), Malvern 2600 and Malvern 3600 series. Such instruments together with their operating manual meet or even exceed the requirements set-out within the ISO 13320 Standard. In a particular embodiment, the particle size is measured using a laser particle size meter, preferably with the powder dispersed in deionized water.

In one embodiment of the pharmaceutical dosage form according to the invention, a fraction of said plurality of particles having a particle size below 160 µm amounts to not more than 95.0 wt.-%, preferably not more than 90 wt.-% of the total weight of said plurality of particles.

In another embodiment of the pharmaceutical dosage form according to the invention, a fraction of said plurality of particles having a particle size above 415 µm amounts to not more than 95.0 wt.-%, preferably not more than 90 wt.-% of the total weight of said plurality of particles.

Certain embodiments of the present invention relate the pharmaceutical dosage form according to the invention for use in the treatment of benign prostatic hyperplasia, wherein preferably the pharmaceutical dosage form is administered orally once daily.

Certain embodiments of the present invention relate to a sachet comprising a pharmaceutical dosage form according to any of the preceding claims.

In one aspect , the sachet comprises the pharmaceutical dosage form and a gas selected from air, nitrogen, oxygen, argon, carbon dioxide and any mixture of the foregoing.

In another aspect, the sachet is made from a material selected from the group consisting of polymers, metal, paper, and any combination thereof; for example, polyethylene, Tyvek®, polypropylene, PET, aluminum, aluminum foil laminates, paper and paper laminates.

In yet another aspect, the sachet is transparent or comprises a transparent portion.

In one embodiment, the sachet is made from a material having a thickness of not more than 1000 µm, more preferably not more than 900 µm, still more preferably not more than 800 µm, yet more preferably not more than 700 µm, even more preferably not more than 600 µm, most preferably not more than 500 µm, and in particular not more than 400 µm.

In another embodiment, the sachet has an inner volume of not more than 1000 cm³, more preferably not more than 500 cm³, still more preferably not more than 250 cm³, yet more preferably not more than 100 cm³, even more preferably not more than cm³ µm, most preferably not more than 75 cm³, and in particular not more than 50 cm³.

Certain embodiments of the present invention relate to a pharmaceutical dosage form comprising either Tamsulosin freebase or a pharmaceutically acceptable salt thereof, especially Tamsulosin hydrochloride, provided in a controlled release matrix, for example, in the form of a sachet, which pharmaceutical dosage form when mixed with water or other suitable liquid is drinkable, for patients with dysphagia. In one embodiment of the present invention Tamsulosin hydrochloride is provided as an extended release suspension for easy administration.

As used herein, the term "controlled release" refers to the release of an agent such as a drug from a composition or dosage form in which the agent is released according to a desired profile over an extended period of time. Controlled release profiles include, for example, extended release, sustained release, prolonged release, pulsatile release, and delayed release profiles.

The present invention also relates in certain embodiments to a controlled release formulation comprising a sachet comprising a unit dosage of a dry powder of Tamsulosin or a pharmaceutically acceptable salt thereof in a controlled release matrix.

The present invention further relates in certain embodiments to a method of treating benign prostatic hyperplasia in a patient suffering therefrom, the method comprising (A) providing a controlled release formulation according to the invention, (B) dispersing said formulation in a liquid to disperse the powder and thereafter (C) orally ingesting the liquid comprising the powder so dispersed.

The present invention further relates to a controlled release formulation according to the invention for use in treating benign prostatic hyperplasia in a patient suffering therefrom by dispersing said formulation in a liquid to disperse the powder and orally ingesting the liquid comprising the powder so dispersed.

The present invention further relates in certain embodiments to a method of treating benign prostatic hyperplasia in a patient suffering therefrom and from dysphagia, the method comprising (A) providing a controlled release formulation according to the invention, (B) dispersing said formulation in a liquid to disperse the powder and thereafter (C) orally ingesting the liquid comprising the powder so dispersed.

The present invention further relates to a controlled release formulation according to the invention for use in treating benign prostatic hyperplasia in a patient suffering therefrom and from dysphagia by dispersing said formulation in a liquid to disperse the powder and orally ingesting the liquid comprising the powder so dispersed.

The present invention further relates in certain embodiments to a method for preparing a controlled release formulation according to the invention comprising (A) preparing a dry powder of Tamsulosin or a pharmaceutically acceptable salt thereof in a controlled release matrix and (B) weighing the powder into a sachet.

The present invention further relates in certain embodiments to a drinkable composition comprising (A) a liquid and (B) a dry powder of Tamsulosin or a pharmaceutically acceptable salt thereof in a controlled release matrix dispersed therein.

The invention will now be described in greater detail with reference to the drawings, wherein:
Fig. 1 is a schematic of an individual particle according to the present invention.

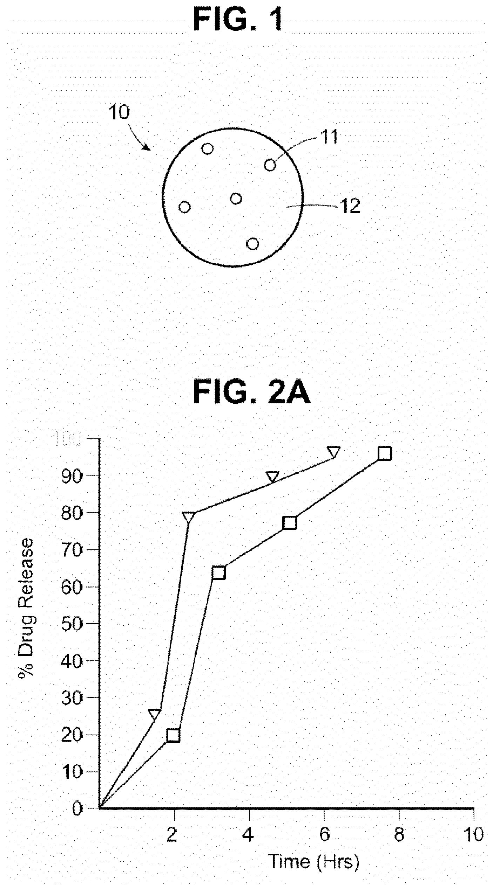

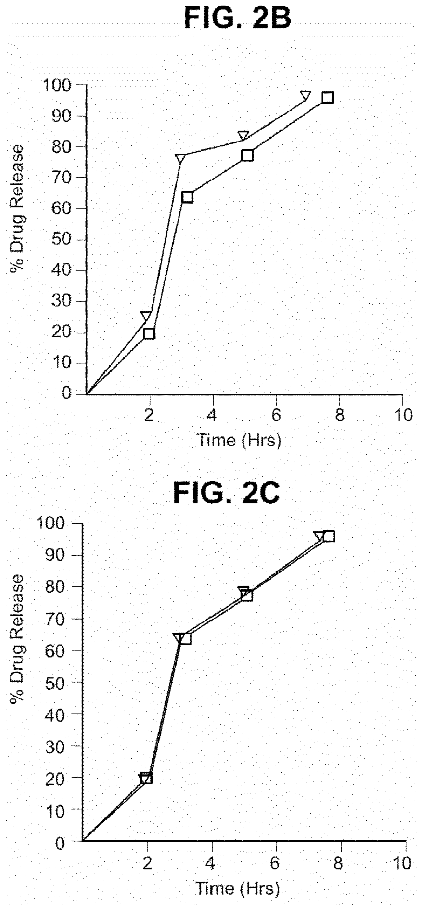

Fig. 2A, 2B and 2C are a series of prophetic graphs each depicting the percentage drug release as a function of time of an FDA-approved dosage form currently approved only in capsule or tablet form (squares) versus one dosage form embodiment according to the invention (triangles). Fig. 2A compares the release profile of the inventive dosage form with the release profile of a first combination of particles (A). Fig. 2B compares the release profiles when in the inventive dosage form the first combination of particles is intentionally supplemented with additional particles (B) to form a second combination of particles (A + B). Fig. 2C compares the release profiles when in the inventive dosage form the second combination of particles is intentionally supplemented with additional particles (C) to form a third combination of particles (A + B + C).
Fig. 3 compares the *in vitro* dissolution of an embodiment of the present invention to FLOMAX®.
Fig. 4 compares the in vivo bioequivalence of an embodiment of the present invention ("fasted" and "fed") to FLOMAX® ("fasted" and "fed").

Exemplary embodiments of the invention relate to formulations containing Tamsulosin or a pharmaceutically acceptable salt thereof, particularly Tamsulosin hydrochloride or another pharmaceutically acceptable salt thereof, designed to provide an extended release oral dosage form that can be easily swallowed by patients with dysphagia. Embodiments of the invention accomplish this with the attributes of the active pharmaceutical ingredient (API) and formulation components, carefully chosen for their contribution to controlled release oral suspension dosage forms. Embodiments of the invention in which a controlled release capsule or tablet dosage form has been replaced with a controlled release suspension offers the advantage that a patient with dysphagia can swallow it. This allows for treatment of a patient that otherwise would not be able to ingest a tablet or capsule. The composition of one exemplary embodiment of the present invention is comprised of a controlled release powder in a unit dose sachet, containing 0.4 mg Tamsulosin hydrochloride, the known therapeutic daily dose for BPH. The composition of another exemplary embodiment of the present invention is comprised of an extended release suspension of 0.4 mg Tamsulosin hydrochloride dispersed in a glass of a suitable liquid, especially water.

In one embodiment, the present invention relates to a pharmaceutical dosage form comprising an amount effective to treat benign prostatic hyperplasia of a combination of a plurality of particles of at least one mixture of (A) Tamsulosin or a physiologically acceptable salt thereof in (B) a controlled release matrix, wherein the combination, when introduced to a quantity of liquid that is thereafter ingested by a patient on an empty stomach, exhibits a controlled release profile over time in the patient substantially identical to FLOMAX® ((R)-5-(2-{[2-(2-Ethoxyphenoxy)ethyl]amino}propyl)-2-methoxybenzene-2-sulfonamide hydrochloride) controlled release tablets or capsules ingested 30 minutes after a meal.

The pharmaceutical dosage form can be in the form of a sachet, as discussed, but can also be in the form of a capsule that can be dissolved in the liquid, for example, a water-soluble capsule, or is breakable or separable into two or more pieces to allow the powdery contents to be poured into the liquid. It is also possible to form a tablet by direct compression of the combination of particles. Although such a tablet, if sufficiently large in size, will not avoid dysphagia, such a tablet should avoid the FLOMAX® food effect.

The amount of Tamsulosin or pharmaceutically acceptable salt thereof can be varied within a wide range, but is most preferably an effective amount to treat BPH, especially a unit dosage of 0.4 mg. Typically, the patient will be administered a single unit dosage of 0.4 mg daily, although a second unit dosage (for a total of 0.8 mg daily) can be administered if needed. Contemplated also is a dosage of Tamsulosin hydrochloride ranging from 0.1 to 1 mg, preferably 0.4-0.8 mg, for the sachet and the other dosage forms described herein.

By "pharmaceutically acceptable salt" is meant all such salts as are conventional in pharmaceutical chemistry and formulation. As used herein, "pharmaceutically acceptable salts" refer to derivatives of the disclosed compounds wherein the parent compound is modified by making salts thereof. Pharmaceutically acceptable salts include salts of acidic (e.g., a carboxylic acid) or basic groups (e.g., a primary, secondary or tertiary amine) present in compounds disclosed herein. Examples of pharmaceutically acceptable salts include, but are not limited to, mineral or organic acid salts of basic residues such as amines; alkali or organic salts of acidic residues such as carboxylic acids; and the like.

The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, lauric, capric, myristic, palmitic, stearic, oleic, linoleic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic acids.

The pharmaceutically acceptable salts of the compounds can be synthesized from the parent compound (e.g., the unprotonated base form of the compound, often referred to as the "free base" of the compound), which contains a basic or acidic moiety, by conventional chemical methods. Generally, such salts can be prepared by reacting the free acid or base forms of these compounds with the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 20th ed., Lippincott Williams & Wilkins, Baltimore, Md., 2000, p. 704, the disclosure of which is hereby incorporated by reference.

Pharmaceutically acceptable salts may also be prepared by reacting the free acid or base forms of compounds with an appropriate base or acid, respectively, in a melt process, optionally in the presence of other pharmaceutically acceptable excipients (e.g., waxes). As used herein, the term "melt process" refers to a process where the free acid or base forms of the compounds are dissolved in one or more excipients that are in molten form (i.e., it is a solid at room temperature) to make a solution wherein the base or acid interacts with the free acid or base form of the compounds, respectively, to form the desired pharmaceutically acceptable salt.

Other salts of the pharmaceutically active agent, which are contemplated by the present invention in order to alter the solubility and/or dissolution rate relative to the parent drug compound (e.g., the free acid or free base form of the compound) include, but are not limited to, pectinate, tannate, phytate, salicylate, saccharinate, acesulfamate, gallate, and terephthalate salts.

A significant side effect of Tamsulosin (and other drugs) is that it leaves a bitter taste in the mouth, which is problematic when contemplating a liquid formulation to be swallowed. Accordingly, in one embodiment of the present invention involves formulating Tamsulosin as the saccharinate (or other sugar salt). We contemplate the specific use of Tamsulosin saccharinate as the active ingredient in any embodiment otherwise described herein. For example, if a specific example relates to Tamsulosin hydrochloride, then the analogous embodiment wherein Tamsulosin saccharinate is substituted for Tamsulosin hydrochloride is likewise set forth by the effect of this paragraph.

Alternatively or in addition to the use of a sugar salt, flavoring agents and/or sweeteners may be utilized. Flavoring agents that may be used in the present invention include, and are not limited to, natural flavors, natural fruit flavors, artificial flavors, artificial fruit flavors, flavor enhancers or mixtures thereof. Natural flavors, artificial flavors or mixtures thereof include, and are not limited to, mint (e.g., peppermint or spearmint), lemon, lime, orange, strawberry, menthol, cinnamon, vanilla, artificial vanilla, chocolate, artificial chocolate or bubblegum. Natural fruit flavors, artificial fruit flavors or mixtures thereof include, and are not limited to, cherry, grape, orange, strawberry or lemon. Flavor enhancers include, and are not limited to, citric acid. Although flavoring agents are generally provided as a minor component and in amounts effective to provide a palatable flavor to the liquid pharmaceutical composition, the addition of at least one flavoring agent is preferred; and, more preferably, up to two flavoring agents may be employed. A flavoring agent used in the inventive formulations in a range of from about 0.01 to about 0.15 grams per 100 mL. The flavorings are generally utilized in amounts that will vary depending upon the individual flavor, and may, for example, range in amounts of about 0.01% to about 10% by weight/weight of the final composition of the sachet.

Examples of sweeteners include sweetening agents, artificial sweeteners and dipeptide based sweeteners, e.g., monosaccharides, disaccharides and polysaccharides such as xylose, ribose, glucose, mannose, galactose, fructose, dextrose, sucrose, sugar, maltose, partially hydrolyzed starch, or corn syrup solids and sugar alcohols such as sorbitol, xylitol, mannitol, saccharin salts, i.e., sodium, or calcium saccharin salts, cyclamate salts, acesulfam-K, ammonium glycyrrhizinate, dipotassium glycyrrhizinate and the free acid form of saccharin L-aspartylphenylalanine methyl ester and mixtures thereof.

Generally, the sweetener will be present in an amount corresponding to about 1 to 60% weight/weight of the total composition of the sachet, the amount depending in part upon whether other sweetener ingredients are present and the level of sweetness desired. Typically when sugar is used, it is present from about 10% to about 50% w/v of the composition. It will be appreciated that combinations of sweeteners can be used. The sweetening agents, when used, may also be used alone or in combination with each other. When an artificial sweetness enhancer is used it may be present in an amount from about 0.05% to about 15% weight/weight of the final composition of the sachet.

Other additives include surfactants, for example, sodium dodecyl sulfate (sodium lauryl sulfate), sorbitan laurate, polyacrylate and salts thereof, for example, sodium polyacrylate, sodium diocotyl sulfosuccinate, sodium oxylate, sodium tartrate, and mixtures thereof, or other surfactants well known to persons skilled in the art; or diluents, for example, hydroxypropyl methyl cellulose, cellulose, talc, starches, for example, corn starch, rice starch, tapioca, wheat starch, potato starch, pre-gelatinized starch, or sodium starch glycolate, or gelatin, and mixtures thereof, or other diluents well known to persons skilled in the art.

In certain embodiments, the Tamsulosin or pharmaceutically acceptable salt thereof is provided in a controlled release formulation in the form of a dry powder for dispersion in a suitable liquid, for example, water, fruit juices (orange juice, apple juice, pineapple juice, etc.). Typically, flavorings, sweeteners, surfactants, diluents and other conventional excipients will be combined in the controlled release formulation as powders themselves.

The Tamsulosin or pharmaceutically acceptable salt is provided in a controlled release matrix that is compatible with the API, is in a form that facilitates suspension of the controlled release formulation in the liquid and slowly releases the API once the liquid containing the suspended controlled release formulation is ingested by the patient.

In a particular embodiment, the controlled release formulation is contained in a sachet. The API and the controlled release matrix will be mixed, preferably, homogeneously, and reduced to a dry powder, preferably by extruding the mixture as an extrudate and thereafter crushing or, preferably, micronizing. The resulting dry powder is then weighed and packaged into sachets, if desired, along with the flavorings, sweeteners, surfactants, diluents and other conventional excipients. The weight is selected so that, for example, the unit dosage of 0.4 mg of Tamsulosin or pharmaceutically acceptable salt is provided per sachet.

Patients are given the sachets with instructions to disperse one sachet each day into a suitable liquid for treatment of BPH (or other disease or disorder). The patient will disperse the sachet into a suitable liquid of their choice, for example, water or fruit juice, and then ingest the resulting suspension. Since the suspension will be easier for patients suffering dysphagia to tolerate, patient compliance should increase over the rates currently observed with Tamsulosin capsules.

Further, since the release from the inventive suspension (or capsules or tablets) does not increase dramatically upon taking proximate to a meal, as is the case with FLOMAX®, the inventive suspension (or capsules or tablets) can be taken on an empty stomach, for example, in the morning, or at bedtime, thereby, also increasing patient compliance and administration flexibility. Indeed, the effect of food on the inventive dosage form is fundamentally different than the effect of food on FLOMAX®. While food intake slows the release of the active ingredient from the inventive dosage forms, in stark contrast to the situation with FLOMAX®, wherein the release rate increases dramatically in the absence of food, the slowing of the release rate in the case of the inventive dosage forms does not constitute a safety concern.

The dry powder of the API and the controlled release matrix will comprise a plurality of particles selected and combined in such a way that the pharmaceutical dosage form when introduced to a quantity of liquid that is thereafter ingested by a patient exhibits a desired controlled release profile of the API in the patient over time. Referring to Fig. 1, there is shown an illustration of one embodiment of a particle 10 according to the present invention. The particle comprises a controlled release matrix 12, throughout which are distributed, preferably homogeneously, molecules of API 11. When these particles are ingested into the patient's body along with the suspending liquid, the API molecules 11 will be released from the controlled release matrix 12.

The factors influencing drug release from matrix materials are well known in the art. Reference is made, for example, to M. Varma et al., "Factors Affecting Mechanism and Kinetics of Drug Release from Matrix-Based Oral Controlled Drug Delivery Systems," Am. J. Drug Deliv., 2(1): 43-57 (2004), the contents of which are incorporated herein by reference. The present invention makes use of the fact that differences in terms of API release rate exist between different particles produced from the same mixture of API + matrix, or between different particles produced from different mixtures of API + matrix, to produce a composite grouping of particles, intentionally individually selected on the basis of their differences in releasing of the API, that collectively combine to release the API in the desired controlled API release rate. In one embodiment, the selection can be as simple as separating off unusually small particles and/or unusually large particles, for example, with the aid of one or more mesh screens, until a combination of particles is obtained that, when combined with other ingredients of the dosage form, for example, sweeteners and other excipients, mimics the release of FLOMAX® in a patient. In another embodiment, individual particles are generated, tested for their release properties alone and in combination with expected co-contents of the sachet, the liquid and in the body and the results are noted and catalogued. Various types of individual particles so catalogued are selected and combined in a way that is calculated to yield the desired release profile, and then this calculation is confirmed by actual testing. Selection and combining individual particles in this way is illustrated in Figs. 2A-2C.

Fig. 2A shows one embodiment of the inventive dosage form with a first combination of particles (A). This first combination of particles (A) may give a release profile (triangles) that is significantly different from that exhibited by the FDA-approved dosage form (squares) under the identical testing conditions. The invention contemplates in this particular embodiment modifying this first combination of particles (A) by intentionally supplementing with additional particles (B) to form a second combination of particles (A + B) that exhibits a release profile that is in closer agreement to that exhibited by the FDA-approved dosage form, as shown in Fig. 2B. Further modifications can be made in the same way until the two release profiles are substantially identical as shown in Fig. 2C. By "substantially identical" is meant at least 80% agreement between the two release profiles, preferably at least 90% agreement, more preferably at least 95% agreement, most preferably at least 99% agreement.

In one embodiment, the pharmaceutical dosage form according to the invention comprises a plurality of particles in a size distribution selected such that the desired release rate over time is achieved when the pharmaceutical dosage form is introduced to the quantity of liquid and ingested by the patient.

In another embodiment, the pharmaceutical dosage form according to the invention comprises a plurality of particles being selected from different mixtures of API and controlled release matrix and combined such that the desired release rate over time is achieved when the pharmaceutical dosage form is introduced to the quantity of liquid and ingested by the patient. In one embodiment, the pharmaceutical dosage form comprises particles of API in a first controlled matrix and also particles of API in a second controlled matrix, wherein the first controlled matrix and the second controlled matrix are different. In a second embodiment, the pharmaceutical dosage form comprises particles of a first concentration of API in a first controlled matrix and also particles of a second concentration of API in a second controlled matrix, wherein the first concentration of API is different from the second concentration of API, and wherein the first controlled matrix is identical to or different from said second controlled matrix.

The pharmaceutical formulations according to the invention do not per se comprise any enteric coating that dissolves as a function of pH. These coatings, which are well known to persons skilled in the art, allow a dosage form even though ingested to pass through the stomach without liberating the active ingredient, which then only or primarily undergoes controlled release in the intestinal tract. Such enteric coatings preferably dissolve at a pH of between 5 and 7. Multiple types of enteric coatings are known in the art and described, for example, in U.S. Patents 5,888,550, 6,139,875, and 8,846,087, and U.S. Patent Applications 2008/0033059, 2012/0082721, and 2013/0115285, all of which are incorporated by reference herein in their entirety.

Although the inventive dosage form does not contain such an enteric coating per see, it may be advantageous to combine enteric coating materials in the controlled release matrix.

These enteric coating materials are preferably selected from the group of shellac, polymethacrylic acid/ethyl acrylate or methacrylic acid/methyl acrylate/methyl methacrylate copolymer, methacrylic acid/methyl methacrylate copolymers, hydroxypropyl methyl cellulose acetate-succinate, cellulose acetate-phthalate, polyvinyl acetate-phthalate, hydroxypropyl methyl cellulose phthalate and/or cellulose acetate-trimellitate.

Further retardation of release over and above that caused in situ, and hence a further modification of the release of the active ingredient can be effected by a variety of methods known to those skilled in the art.

Matrix materials which can be used for instant invention include physiologically compatible, hydrophilic materials known to those skilled in the art, e.g. Bauer, et al., "Überzogene Arzneiformen" ("Coated Pharmaceutical Forms"), Wissenschaftliche Verlagsgesellschaft mbH Stuttgart, 1988, p. 69 et seq., which is incorporated herein by reference and thus forms part of this disclosure. The hydrophilic matrix materials used are preferably polymers and particularly preferably cellulose ethers, cellulose esters and/or acrylic resins, preferably poly(meth)acrylates. The matrix materials used are very particularly preferably ethyl cellulose, hydroxypropyl methyl cellulose, hydroxypropyl cellulose, hydroxymethyl cellulose, poly(meth)acrylic acid and/or derivatives thereof such as their salts, amides or esters.

Other matrix materials include hydrophobic materials such as hydrophobic polymers, waxes, fats, long-chain fatty acids, fatty alcohols or corresponding esters or ethers, or mixtures thereof. The hydrophobic materials used are particularly preferably C12-C30 fatty acid mono- or diglycerides and/or C12-C30 fatty alcohols and/or waxes, or mixtures thereof.

Materials suitable for the sustained-release matrix also include polyalkylene oxides, preferably, polyethylene oxide; polyvinyl acetates; and polyvinyl pyrrolidones.

The sustained-release matrix material used can also be mixtures of said hydrophilic and hydrophobic materials.

The matrix components will make up the bulk of the inventive dosage form. For a unit dosage form containing the preferred 0.4 mg of Tamsulosin, the amount of the matrix components will total from 50-1000 mg, preferably 75-500 mg, most preferably 100-400 mg.

To adjust the rate of release of the active substances, the inventive formulations can optionally contain, in addition to the water-insoluble polymers, non-retarding, preferably water-soluble polymers in amounts of up to 30 wt. %, such as polyvinylpyrrolidone; or water-soluble celluloses, preferably hydroxypropyl methyl cellulose or hydroxypropyl cellulose; and/or hydrophilic pore-forming agents such as sucrose, sodium chloride or mannitol; and/or plasticizers known in the art.

To further retard the release of the active ingredient, the pharmaceutical formulations according to the invention can preferably also contain the active ingredient uniformly distributed in a sustained-release matrix.

In one embodiment, the controlled release material comprises at least one material selected from the group consisting of ethyl cellulose, hydroxypropyl methyl cellulose, and derivatives thereof.

In another embodiment, the controlled release material comprises a mixture of (i) ethyl cellulose or a derivative thereof, and (ii) hydroxypropyl methyl cellulose or a derivative thereof.

In another embodiment, the ethyl cellulose or derivative thereof is selected from the group consisting of ethyl cellulose, and blends of ethyl cellulose and cellulose acetate phthalate; and the hydroxypropyl methyl cellulose or derivative thereof is selected from the group consisting of hydroxypropyl methyl cellulose, hydroxypropyl methyl cellulose acetate succinate, and hydroxypropyl methyl cellulose phthalate.

Particle size and matrix composition are factors influencing the release profile of the active ingredient from the inventive formulations. Particle sizes range from 1-1000 µm, especially, 1-500 µm, preferably <10-400 µm, most preferably <10-350 µm. Smaller sized particles within these ranges may improve the aesthetics of the dispersed products, for example, as explained in U.S. Patent No. 5,149,541, which pertains to METAMUCIL® (psyllium husk). A combination of particles ranging in size between 100-600 µm, preferably 125-500 µm, most preferably 150-450 µm provides especially good results where the matrix is based on cellulose derivatives, particularly ethyl cellulose and/or hydroxypropyl methyl cellulose, and acid esters thereof.

In a particular embodiment, the inventive dosage form is prepared by (A) extruding an extrudate comprising: a mixture of (i) Tamsulosin or a salt thereof, (ii) hydroxypropyl methyl cellulose or a derivative thereof, and (iii) ethyl cellulose or a derivative thereof, (B) reducing the extrudate to a powder, (C) selecting from said powder particles of various sizes to give a combination of selected particles exhibiting a distribution of particle sizes such that when the combination of selected particles is ingested by a patient on an empty stomach along with a quantity of liquid and optionally excipients there is produced in said patient an in vivo release profile of Tamsulosin or salt thereof over the course of a given period of time that substantially mimics the in vivo release profile over the same period of time of FLOMAX® ingested by said patient 30 minutes after a meal.

Thus, another particular embodiment of the invention provides a pharmaceutical composition in the form of a powder, said powder comprising a plurality of particles, wherein each of the particles consists of:
(a) Tamsulosin or a pharmaceutically acceptable salt thereof; in
(b) a controlled release matrix consisting of:
   (i) ethyl cellulose or a derivative thereof; and
   (ii) hydroxypropylmethylcellulose or a derivative thereof;
wherein the particles do not comprise an enteric coating; and
wherein the particles range in size from 100-600 µm.

A brief scheme for adapting any suitable active ingredient to the present invention is shown below for an extruded product.

Brief Scheme:
Development steps for Particulate component of Sachet:
Establish the matrix compatibilities with Tamsulosin HCl.

First Tier Alternative matrix components; 50 to 80%VA64 (vinylpyrrolidone-vinyl acetate); 2 to 10% PEG1500 (polyethylene glycol 500)/40 to 90%VA64/10% to 40%HPMCs; 10% PEG1500/40%VA64; 10% TPGS (Tocopherol propylene glycol succinate)/40 to 10% TPGS/40%VA64; HPMCAS MF (hydroxypropylmethylcellulose acetate-succinate); Eudragit L30; Eudragit RS PO; HPMCT (hydroxypropylmethylcellulose trimellitate).

Second Tier Alternative matrix components; Methacrylic acid copolymer type A, NF; Methacrylic acid copolymer type B, NF; Methacrylic acid copolymer type C, NF; Polyethylene oxide; Polyvinylpyrrolidone; Ethylene vinyl acetate; Poly(L-lactic-co-glucolic acid); Polycaprolate; Microcrystalline wax; Xanthum gum; Propylene glycol; Ethyl cellulose; Glycerol distearate; Glycerol tristearate; Glycerol dioleate; Glycerol trioleate. Matrix Diluents: Docusate sodium; Xanthum gum; Polysorbate 80; Microcrystalline cellulose; Polyethylene glycols; Matrix Lubricants; Stearic acid; Sodium lauryl sulfate; Starch; Pregelatinized starch; talc.

The mixture of matrix components will be extruded or spheronized. Prepare the particulates in size ranges: generate separate milled particle fractions; of <10 micron, 10 to 20 micron, 20 to 30 micron, 30 to 40 micron, 40 to 50 micron, 50 to 60 micron and 60 to 70 micron.

Blend the particle size fractions to establish an appropriate particle size mixture.

### Development of Sachet mixture:

Add surfactant, diluent powder, and sweetener; Surfactants and/or dispersing agent; Sodium dodecyl sulfate (Sodium lauryl sulfate); Sorbitan laurate; Polyacrylate; Sodium diocotyl sulfosuccinate; Sodium oxylate; Sodium tartrate; Diluent: HPMC; Cellulose; Talc; Corn Starch; Rice Starch; Tapioca; Wheat starch; Potato starch; Pre-gelatinized starch; Sodium starch glycolate; Gelatin; Flavorings (GRAS agents); Mannitol; Sorbitol; Strawberry flavor; Lemon flavor; Lime flavor; Sucralose.

Blend the surfactant, diluent powder and sweetener.

Characterize the sachet in a diluent for drinking.

Prepare various slurries compatible with sachet components; Slurry in 8 oz water; Slurry in 8 oz juice; Orange; Cranberry; Tomato; grapefruit; Slurry in 8 oz apple sauce.

Characterize per target product profile (See Table 1); Potency; Drug release profile; Impurities;

**Table 1: Development Target Product Profile for Powder for Oral Suspension**

| DTPP Parameter | Target |
|---|---|
| Name | Tamsulosin HCl Sachet |
| API | Tamsulosin HCl |
| Dosage Strengths | 0.4 mg, once daily |
| Target Patient Population | Adult male |
| Pediatric or Gender Use | Not for female use, not indicated for pediatric use. No overall differences in efficacy or safety |
| Indication | Tamsulosin is an alpha1 adrenoreceptor indicated for treatment of the signs and symptoms of benign prostatic hyperplasia |
| Schedule Class | N/A |
| Dosage | Sachets (∼200 mg or less), single use pouch |
| | "Reconstitute in water or juice (8 oz) and drink" to be modified as appropriate |
| Container closure system | Aluminum pouch |
| Storage | Store at Controlled Room Temperature encompassing 20°- 25°C (68° to 77°F), allowing for excursions between 15°-30°C (59°F-86°F) per USP General Notices |
| Appearance | Free flowing particles |
| Potency | Release: 90.0% to 110.0% |
| | Stability: 90.0% to 110.0% |
| Uniformity of Dosage Units | Complies with requirements of USP <905> |
| Dissolution | Complies with USP <711> dissolution testing requirements for Tamsulosin (capsule USP monograph Test 3); 8 hr total test time |
| | Acid stage: 2 hr in 0.003% polysorbate 80 pH 1.2 (500 mL) 100 rpm |
| | Buffer stage: 6 hr total, phosphate buffer pH 7.2 |
| | At 2 hr; NMT 20% |
| | At 3hr; 50% to 85% dissolved |
| | At 8 hr: NLT 80% dissolved |
| | (See USP tamuslosin capsule test 3 for method description) |
| Impurities | Complies with ICH and/or FDA requirements for impurities in drug products |
| Residual Solvents | Controlled per USP<467> per each supplier's statement for components. None added during processing for drug product |
| Microbial evaluation | Meets USP acceptance criteria for non-aqueous preparations for oral use |
| Stability | Shelf life ≥ 12 months, prefer 24 to 36 months |

| | |
|---|---|
| DTPP=development target product profile | |

Drug release method: Use USP method for Tamsulosin HCl tablets. Compare experimental formulation with LD in various media. Stage 1: 2 hours in acidic buffer: 0.003% polysorbate 80 in pH 1.2 (using HCl); Use Apparatus 2 for tablets (for reference), no chamber required for sachet; 100 rpm; Test time 0, 30 min, 1h, 1.5h and 2 h. Followed by Stage 2: dissolution profile in phosphate buffer pH 7.2; Use Apparatus 2 for tablets (for reference) no chamber required for sachet; 100 rpm; Test 0, 1 h, 2h, 3h, 4h, 5h, and 6 h. Additional dissolution profiles generated in pH 1.2, 4.5 and 6.8 medium per the SUPAC-MR Guidance requirements. Equivalency will be established by achieving multi-point dissolution similarity (f2) values of >50 with results obtained for the experimental formulation and the LD. The optimum formulation will show dissolution similarity (f2) values of >50 when compared to the LD dissolution profile.

Particular embodiments Emb. 1 to Emb. 49 of the invention are as follows: Emb. 1: A pharmaceutical dosage form comprising a plurality of particles providing controlled release of Tamsulosin or a physiologically acceptable salt thereof; wherein each of said particles comprises a controlled release matrix in which the Tamsulosin or the physiologically acceptable salt thereof is embedded; wherein said controlled release matrix comprises a mixture of a first cellulose derivative and a second cellulose derivative; wherein said first cellulose derivative differs from said second cellulose derivative; wherein said first cellulose derivative and said second cellulose derivative each are independently of one another selected from the group consisting of cellulose ethers and cellulose esters; and wherein the total content of said first cellulose derivative and said second cellulose derivative is at least 50 wt.-%, relative to the total weight of the plurality of particles; and wherein the particles are not coated with an enteric coating material. Emb. 2: The pharmaceutical dosage form according to Emb. 1, wherein the particles have an average particle size within the range of from 160 µm to 415 µm, preferably from 175 µm to 400 µm. Emb. 3: The pharmaceutical dosage form according to Emb. 1 or 2, wherein (i) a fraction of said plurality of particles having a particle size below 160 µm amounts to not more than 95.0 wt.-%, preferably not more than 90 wt.-% of the total weight of said plurality of particles; or (ii) a fraction of said plurality of particles having a particle size above 415 µm amounts to not more than 95.0 wt.-%, preferably not more than 90 wt.-% of the total weight of said plurality of particles. Emb. 4: The pharmaceutical dosage form according to any of the preceding Embs., wherein the total content of said first cellulose derivative and said second cellulose derivative is at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%, relative to the total weight of the plurality of particles. Emb. 5: The pharmaceutical dosage form according to any of the preceding Embs., wherein (i) the content of said first cellulose derivative is at least 10 wt.-%, more preferably at least 15 wt.-%, still more preferably at least 20 wt.-%, yet more preferably at least 25 wt.-%, even more preferably at least 30 wt.-%, most preferably at least 35 wt.-%, and in particular at least 40 wt.-%, relative to the total weight of the plurality of particles; and/or (ii) the content of said second cellulose derivative is at least 10 wt.-%, more preferably at least 15 wt.-%, still more preferably at least 20 wt.-%, yet more preferably at least 25 wt.-%, even more preferably at least 30 wt.-%, most preferably at least 35 wt.-%, and in particular at least 40 wt.-%, relative to the total weight of the plurality of particles; and/or (iii) the content of Tamsulosin or the physiologically acceptable salt thereof is at least 3.0 wt.-%, more preferably at least 4.0 wt.-%, still more preferably at least 5.0 wt.-%, yet more preferably at least 6.0 wt.-%, even more preferably at least 7.0 wt.-%, most preferably at least 8.0 wt.-%, and in particular at least 9.0 wt.-%, relative to the total weight of the plurality of particles. Emb. 6: The pharmaceutical dosage form according to any of the preceding Embs., wherein (i) the content of said first cellulose derivative is within the range of 45±40 wt.-%, preferably 45±35 wt.-%, more preferably 45±30 wt.-%, still more preferably 45±25 wt.-%, yet more preferably 45±20 wt.-%, even more preferably 45±15 wt.-%, most preferably 45±10 wt.-%, and in particular 45±5 wt.-%, relative to the total weight of the plurality of particles; and/or (ii) the content of said second cellulose derivative is within the range of 45±40 wt.-%, preferably 45±35 wt.-%, more preferably 45±30 wt.-%, still more preferably 45±25 wt.-%, yet more preferably 45±20 wt.-%, even more preferably 45±15 wt.-%, most preferably 45±10 wt.-%, and in particular 45±5 wt.-%, relative to the total weight of the plurality of particles; and/or (iii) the content of the Tamsulosin or the physiologically acceptable salt thereof is within the range of 10±8 wt.-%, preferably 10±7 wt.-%, more preferably 10±6 wt.-%, still more preferably 10±5 wt.-%, yet more preferably 10±4 wt.-%, even more preferably 10±3 wt.-%, most preferably 10±2 wt.-%, and in particular 10±1 wt.-%, relative to the total weight of the plurality of particles, relative to the total weight of the plurality of particles. Emb. 7: The pharmaceutical dosage form according to any of the preceding Embs., wherein the relative weight ratio of said first cellulose derivative to said second cellulose derivative is within the range of from 5:1 to 1:5; preferably from 4.5:1 to 1:4.5; more preferably from 4:1 to 1:4, still more preferably from 3.5:1 to 1:3.5, yet more preferably from 3:1 to 1:3, even more preferably from 2.5:1 to 1:2.5, most preferably from 2:1 to 1:2, and in particular from 1.5:1 to 1:1.5. Emb. 8: The pharmaceutical dosage form according to any of the preceding Embs., wherein said first cellulose derivative and said second cellulose derivative each are independently of one another selected from the group consisting of ethyl cellulose, cellulose acetate phthalate, hydroxypropyl methyl cellulose, hydroxypropyl methyl cellulose acetate succinate, and hydroxypropyl methyl cellulose phthalate. Emb. 9: The pharmaceutical dosage form according to any of the preceding Embs., wherein (i) the first cellulose derivative is ethyl cellulose; and/or (ii) the second cellulose derivative is selected from the group consisting of hydroxypropyl methyl cellulose, hydroxypropyl methyl cellulose acetate succinate, and hydroxypropyl methyl cellulose phthalate. Emb. 10: The pharmaceutical dosage form according to any of the preceding Embs., wherein said plurality of particles (i) are free flowing, non-compacted particles; and/or (ii) have a particle size in the range of from 100 µm to 600 µm, preferably 150 µm to 450 µm. Emb. 11: The pharmaceutical dosage form according to any of the preceding Embs., wherein said plurality of particles (i) are not coated with any coating material; and/or (ii) do not contain one or more materials selected from the group consisting of microcrystalline cellulose, polyethylene oxide, alginic acid, alginate salts, lactose, and glycerol behenate; preferably do not contain any of the foregoing materials; and/or (iii) essentially consists of, preferably consist of said first cellulose derivative, said second cellulose derivative and Tamsulosin or the physiologically acceptable salt thereof. Emb. 12: The pharmaceutical dosage form according to any of the preceding Embs., for use in the treatment of benign prostatic hyperplasia. Emb. 13: The pharmaceutical dosage form for use according to Emb. 12, wherein the pharmaceutical dosage form is administered orally once daily. Emb. 14: A sachet comprising a pharmaceutical dosage form according to any of the preceding Embs. Emb. 15: The sachet according to Emb. 13, wherein the sachet (i) comprises the pharmaceutical dosage form and a gas selected from air, nitrogen, oxygen, argon, carbon dioxide and any mixture of the foregoing; and/or (ii) is made from a material selected from the group consisting of polymers, metal, paper, and any combination thereof; and/or (iii) is transparent or comprises a transparent portion; and/or (iv) is made from a material having a thickness of not more than 1000 µm; and/or (v) has an inner volume of not more than 1000 cm³. Emb. 16: A pharmaceutical dosage form, preferably according to any of Embs. 1 to 15, wherein said dosage form comprises an amount effective to treat benign prostatic hyperplasia of a combination of a plurality of particles, wherein each of said particles comprises a mixture of (A) Tamsulosin or a physiologically acceptable salt thereof in (B) a controlled release matrix, wherein the combination of particles, when introduced to a quantity of liquid that is thereafter ingested by a patient on an empty stomach, exhibits a controlled release profile over time in the patient substantially identical to FLOMAX® ((R)-5-(2-{[2-(2-Ethoxyphenoxy)ethyl]amino}propyl)-2-methoxybenzene-1-sulfonamide hydrochloride) controlled release tablets or capsules ingested 30 minutes after a meal. Emb. 17: The pharmaceutical dosage form according to any of the preceding Embs., which comprises Tamsulosin HCl. Emb. 18: The pharmaceutical dosage form according to Emb. 17, which comprises 0.4 mg of Tamsulosin HCl. Emb. 19: The pharmaceutical dosage form according to any of the preceding Embs., wherein the controlled release matrix comprises a material selected from the group consisting of hydrophilic materials, hydrophobic materials, and mixtures thereof. Emb. 20: The pharmaceutical dosage form according to Emb. 19, wherein the controlled release matrix comprises a material selected from the group consisting of cellulose ethers, cellulose esters, acrylic resins, hydrophobic polymers, waxes, fats, long-chain fatty acids, fatty alcohols, fatty alcohol esters, fatty alcohol ethers, polyalkylene oxides, polyvinyl acetates, polyvinyl pyrrolidones, and mixtures thereof. Emb. 21: The pharmaceutical dosage form according to Emb. 20, wherein the controlled release matrix comprises at least one material selected from the group consisting of ethyl cellulose, hydroxypropyl methyl cellulose, hydroxypropyl cellulose, hydroxymethyl cellulose, poly(meth)acrylic acid, and derivatives thereof. Emb. 22: The pharmaceutical dosage form according to Emb. 21, wherein the controlled release material comprises at least one material selected from the group consisting of ethyl cellulose, hydroxypropyl methyl cellulose, and derivatives thereof. Emb. 23: The pharmaceutical dosage from according to Emb. 22, wherein the controlled release material comprises a mixture of (i) ethyl cellulose or a derivative thereof, and (ii) hydroxypropyl methyl cellulose or a derivative thereof. Emb. 24: The pharmaceutical dosage form according to Emb. 23, wherein the ethyl cellulose or derivative thereof is selected from the group consisting of ethyl cellulose, and blends of ethyl cellulose and cellulose acetate phthalate; and the hydroxypropyl methyl cellulose or derivative thereof is selected from the group consisting of hydroxypropyl methyl cellulose, hydroxypropyl methyl cellulose acetate succinate, and hydroxypropyl methyl cellulose phthalate. Emb. 25: The pharmaceutical dosage form according to any of Embs. 16 to 24, wherein the at least one mixture of (A) and (B) is homogeneous. Emb. 26: The pharmaceutical dosage form according to any of Embs. 16 to 25, wherein the amount of (A) is 0.4 mg and the amount of (B) ranges from 100 mg to 400 mg in each of said at least one mixtures. Emb. 27: The pharmaceutical dosage form according to any of the preceding Embs., which comprises said plurality of particles in a size distribution selected such that said release rate over time is achieved when the pharmaceutical dosage form is introduced to said quantity of liquid and ingested by said patient. Emb. 28: The pharmaceutical dosage form according to Emb. 27, wherein the size distribution ranges from about 100 µm to about 600 µm, preferably from about 150 µm to about 450 µm. Emb. 29: The pharmaceutical dosage form according to any of the preceding Embs., which exhibits a controlled release rate over time in the patient that at any given time after ingestion is at least 80% in agreement, preferably at least 90% in agreement, with the controlled release rate of FLOMAX® ((R)-5-(2-{[2-(2-Ethoxyphenoxy)ethyl]amino}propyl)-2-methoxybenzene-1-sulfonamide hydrochloride) in the patient at the same given time after ingestion. Emb. 30: The pharmaceutical dosage form according to any of the preceding Embs., which comprises said plurality of particles being selected from different mixtures of (A) and (B) and combined such that said release rate over time is achieved when the pharmaceutical dosage form is introduced to said quantity of liquid and ingested by said patient. Emb. 31: The pharmaceutical dosage form according to Emb. 30, which comprises particles of (A) in a first controlled matrix (B) and also particles of (A) in a second controlled matrix (B), wherein said first controlled matrix (B) and said second controlled matrix (B) are different. Emb. 32: The pharmaceutical dosage form according to Emb. 30, which comprises particles of a first concentration of (A) in a first controlled matrix (B) and also particles of a second concentration of (A) in a second controlled matrix (B), wherein said first concentration of (A) is different from said second concentration of (A), and wherein said first controlled matrix (B) is identical to or different from said second controlled matrix (B). Emb. 33: The pharmaceutical dosage form according to any of the preceding Embs., which is in the form of a sachet. Emb. 34: The pharmaceutical dosage form according to any of the preceding Embs., which further comprises at least one substance selected from the group consisting of sweeteners, surfactants and enteric release materials. Emb. 35: The pharmaceutical dosage form according to any of the preceding Embs., which comprises the at least one substance selected from the group consisting of sweeteners, surfactants and enteric release materials in the form of a powder or granules. Emb. 36: The pharmaceutical dosage form according to any of the preceding Embs., which is in the form of a capsule. Emb. 37: The pharmaceutical dosage form according to Emb. 36, wherein the capsule has a casing that dissolves in liquid. Emb. 38: The pharmaceutical dosage form according to Emb. 36 or 37, wherein the capsule has a casing that is breakable or separable. Emb. 39: The pharmaceutical dosage form according to any of the preceding Embs., which further comprises at least one substance selected from the group consisting of sweeteners, surfactants and enteric release materials. Emb. 40: The pharmaceutical dosage form according to any of the preceding Embs., which comprises the at least one substance selected from the group consisting of sweeteners, surfactants and enteric release materials in the form of a powder or granules. Emb. 41: The pharmaceutical dosage form according to any one of Embs. 1 to 35, which is in the form of a tablet. Emb. 42: The pharmaceutical dosage form according to any of the preceding Embs., which further comprises a 5α-reductase inhibitor. Emb. 43: The pharmaceutical dosage form according to Emb. 42, wherein the 5α-reductase inhibitor is dutasteride or a pharmaceutically acceptable salt thereof. Emb. 44: The pharmaceutical dosage form according to any one of Embs. any of the preceding Embs., wherein the at least one mixture of (A) in (B) is a crushed or micronized extrudate. Emb. 45: A pharmaceutical dosage form comprising a combination of a plurality of particles from at least one extrudate of a homogeneous mixture of (A) Tamsulosin or a physiologically acceptable salt thereof in (B) a controlled release matrix, wherein the combination of particles, when introduced to a quantity of liquid that is thereafter ingested by a patient on an empty stomach, exhibits a controlled release profile over time in the patient substantially identical to FLOMAX® ((R)-5-(2-{[2-(2-Ethoxyphenoxy)ethyl]amino}propyl)-2-methoxybenzene-1-sulfonamide hydrochloride) controlled release tablets or capsules ingested 30 minutes after a meal. Emb. 46: A method of treating benign prostatic hyperplasia in a patient suffering therefrom, said method comprising (A) providing a pharmaceutical dosage form according to any of the preceding Embs., (B) dispersing the pharmaceutical dosage form in a liquid to disperse the plurality of particles in the liquid and thereafter (C) orally ingesting the liquid comprising the plurality of particles so dispersed. Emb. 47: A method of preparing a pharmaceutical dosage form according to any of the preceding Embs., said method comprising: (a) preparing at least one mixture of (A) Tamsulosin or a physiologically acceptable salt thereof in (B) a controlled release matrix; (b) reducing the at least one mixture to a plurality of particles; and (c) selecting particles obtained in (B) and combining the particles in such a way that a resulting combination of particles, when introduced to a quantity of liquid that is thereafter ingested by a patient on an empty stomach, exhibits a controlled release profile over time in the patient substantially identical to FLOMAX® ((R)-5-(2-{[2-(2-Ethoxyphenoxy)ethyl]amino}propyl)-2-methoxybenzene-1-sulfonamide hydrochloride) controlled release tablets or capsules ingested 30 minutes after a meal. Emb. 48: A method of preparing a pharmaceutical dosage form according to any of the preceding Embs., said method comprising: (a) extruding from an extruder at least one extrudate of a homogeneous mixture of (A) Tamsulosin or a physiologically acceptable salt thereof in (B) a controlled release matrix; (b) reducing the at least one extrudate to a plurality of particles; and (c) combining particles obtained in (B) from one or more extrudates in such a way that a resulting combination of particles, when introduced to a quantity of liquid that is thereafter ingested by a patient on an empty stomach, exhibits a controlled release profile over time in the patient substantially identical to FLOMAX® ((R)-5-(2-{[2-(2-Ethoxyphenoxy)ethyl]amino}propyl)-2-methoxybenzene-1-sulfonamide hydrochloride) controlled release tablets or capsules ingested 30 minutes after a meal. The invention will now be described in greater detail with reference to the following non-limiting examples. Emb. 49: A pharmaceutical dosage form comprising a plurality of particles providing controlled release of Tamsulosin or a physiologically acceptable salt thereof; wherein each of said particles comprises a controlled release matrix in which the Tamsulosin or the physiologically acceptable salt thereof is embedded; wherein said controlled release matrix comprises a mixture of a first cellulose and a second cellulose; wherein said first cellulose differs from said second cellulose; and wherein the total content of said first cellulose and said second cellulose is at least 50 wt.-%, relative to the total weight of the plurality of particles; and wherein the particles are not coated with an enteric coating material.

Example 1: (Prophetic): The following components, in the percents by weight indicated, are homogeneously mixed in a mixer, for example a roller mixer, a shaking mixer, a shearing mixer or a compulsory mixer:

| | |
|---|---|
| Tamsulosin hydrochloride | 0.1-10% |
| Polyethylene glycol | 30-80% |
| Sodium lauryl sulfate | 1-10% |
| Pregelatinized starch | 1-5% |
| Microcrystalline cellulose | 5-20% |

(Percents given are by weight, based on the total weight of the formulation.)

The mixture is extruded through a twin screw extruder, for example, the twin screw extruder available from the company Leistritz (Numberg) of type ZSE 18 HP 40D, preferably with screws equipped with eccentric screw ends. A heatable nozzle plate with 8 orifices each having a diameter of 1.0 mm can be used as the nozzle. The extrusion parameters can be set, for example at the following values: screw speed: 150 rpm; throughput: 2 kg/h; product temperature: 60°C. to 140°C., preferably 80°C. to 140°C., most preferably 110°C. to 140°C. with corresponding barrel temperature.

The rod-shaped extrudate is cut at predetermined lengths and the cut pieces are reduced to powder by micronization in suitable equipment. The powder particles are separated according to size and, based on knowledge of the release properties of individual particles, selected on this basis and recombined into a new powder expected to have a desired release profile of API.

The powder is weighed out and along with flavorings, surfactants and sweeteners filled into sachets each containing a unit dosage of 0.4 mg of Tamsulosin hydrochloride.

Example 2: (Prophetic): The following components, in the percents by weight indicated, are homogeneously mixed in a mixer, extruded, cut and micronized in a manner analogous to Example 1:

| | |
|---|---|
| Tamsulosin hydrochloride | 0.1-10% |
| Hydroxymethyl propyl cellulose | 30-80% |
| Sodium lauryl sulfate | 1-10% |
| Pregelatinized starch | 1-5% |
| Microcrystalline cellulose | 5-20% |

The powder particles are separated according to size and, based on knowledge of the release properties of individual particles, selected on this basis and recombined along with desired particles from Example 1 into a new powder expected to have a desired release profile of API.

The powder is weighed out and along with flavorings, surfactants and sweeteners filled into sachets each containing a unit dosage of 0.4 mg of Tamsulosin hydrochloride.

Example 3: (Prophetic): A crystalline active pharmaceutical ingredient extruded with a carrier and release controlling agent, milled and packaged in a sachet.

| | |
|---|---|
| Tamsulosin saccharinate, micronized | 0.1-10% |
| Polyethylene glycol | 30-80% |
| Sodium lauryl sulfate | 1-10% |
| Pregelatinized starch | 1-5% |
| Microcrystalline cellulose | 5-20% |
| Flavoring | 5-30% |

(Percents given are by weight, based on the total weight of the formulation.)

Example 4: (Actual): The following ingredients were combined as a pre-extrusion blend:

| | |
|---|---|
| Tamsulosin HCl | 10 wt.% |
| Ethocel Standard 10 Premium | 45 wt.% |
| Hypromellose Acetate Succinate, NF, AS-MMP Grade | 45 wt.% |

| | |
|---|---|
| * All wt.% based on the total weight of the pre-extrusion blend | |

The pre-extrusion blend was introduced to the hopper and extruded through the die of a Leistritz (Numberg, Germany) ZSE 18 HP 40:1 Twin Screw extruder operating at a screw speed of 100±10 RPM; throughput: 0.5±0.2 kg/hr; melt plate temperature: 160°C±5°C; heating zone temperature: 160°C±5°C; and die heater temperature: 160°C±5°C. The extrudate was extruded onto a conveyor belt and cooled. The cooled extrudate was milled using a Fitzmill with a 0.033" perforated screen with the blades in the hammer position, until a powder was obtained. This powder was subjected to 40-mesh and 100-mesh screens to eliminate fines and coarse particles, and to yield a particle size range for the resulting powder between 150-425 µm.

A portion of this powder was then combined with dry excipients into a sachet having the following formulation:

| | % | mg/sachet |
|---|---|---|
| Tamsulosin HCl | 1.1 | 0.452 |
| Ethocel Standard 10 Premium | 5 | 2 |
| Hypromellose Acetate Succinate, NF, AS-MMP Grade | 5 | 2 |
| Avicel | 85.9 | 34.8 |
| Flavor | 1 | 0.4 |
| Sucralose | 1 | 0.4 |
| sodium stearyl fumarate | 0.5 | 0.4 |
| silicon dioxide | 0.5 | 0.4 |
| Total | 100 | 40.512 |

Example 5: (Prophetic): The formulation of Example 4 is, instead of being combined into a sachet, combined into a water-soluble, two-part capsule. The contents can be introduced to a liquid, for example, water or juice, simply by dropping the entire capsule into the liquid, or by separating the two parts of the capsule and pouring the capsule contents into the liquid.

Example 6: (Prophetic): The formulation of Example 4 is, instead of being combined into a sachet, pressed on a tablet-press into a round or other-shaped pill. The pill can be swallowed in the same manner as FLOMAX® capsules. However, because the inventive capsules have a different composition, they should be free from the FLOMAX® capsule food effect.

### Example 7: (Actual): Dissolution Studies

Objective: The primary objective of this study was to establish by HPLC the similarity of the *in vitro* profile of the sachet formulation of Example 4 compared to the *in vitro* profile of FLOMAX® capsules when measured according to USP <711> dissolution testing requirements for Tamsulosin (capsule USP monograph Dissolution Test 3): 8 hr total test time; Acid stage: 2 hr in 0.003% polysorbate 80 pH 1.2 (500 mL) 100 rpm; Buffer stage: 6 hr total, phosphate buffer pH 7.2; At 3 hr: 65% to 85% dissolved; and At 8 hr: NLT 80% dissolved.

Protocol: Dissolution Parameters; USP Apparatus: USP Apparatus II (Paddle); Rotation Speed: 100 RPM; Sample Volume: 5 mL with no media replacement; Sinkers/Baskets: None; Filters: 0.45 µm nylon. Bath Temperature: 37°C±0.5°C; Pull Times: 2 hrs, 3 hrs & 8 hrs.

Acid Stage: 500 mL of acid stage media was added to each dissolution vessel, and allowed to equilibrate to 37°C±0.5°C. Each formulation was weighed and the whole contents removed and sprinkled carefully into each of six (6) dissolution vessels and the timer was started. At 2 hours, a single 5 mL sample was removed from each vessel using a cannula. The sample was taken from the zone midway between the surface of the dissolution medium and the top of the paddle blade and not less than 1 cm from the vessel wall and shaft. The sample was filtered directly to a glass HPLC vial using a 0.45 µm nylon membrane.

Dissolution: 500 mL of pre-warmed (to 37°C) buffer stage concentrate was added into each of the six (6) dissolution vessels containing the acid stage media. The total volume was then 1000 mL and had a pH of 7.2. The timer was allowed to continue. At 3 hours and 8 hours, a single 5 mL sample from each vessel was removed using a cannula. The sample was taken from the zone midway between the surface of the dissolution medium and the top of the paddle blade and not less than 1 cm from the vessel wall and shaft. The sample was filtered directly to a glass HPLC vial using a 0.45 µm nylon membrane. The samples would expire in three (3) days stored at ambient laboratory conditions.

HPLC Parameters: Instrument: Waters Alliance™ 2695 HPLC System; Column: Atlantis, dC18, 5µm, 4.6 x 150 mm; Flow Rate: 1.2 mL/min; Run Time: 10 min; Sample Temp: Ambient; Column Temp: 40°C; Injection Volume: 100 µL; Detection: 225 (nm); Pump: Isocratic; Needle Wash/Seal Wash: Acetonitrile/Water (50/50).

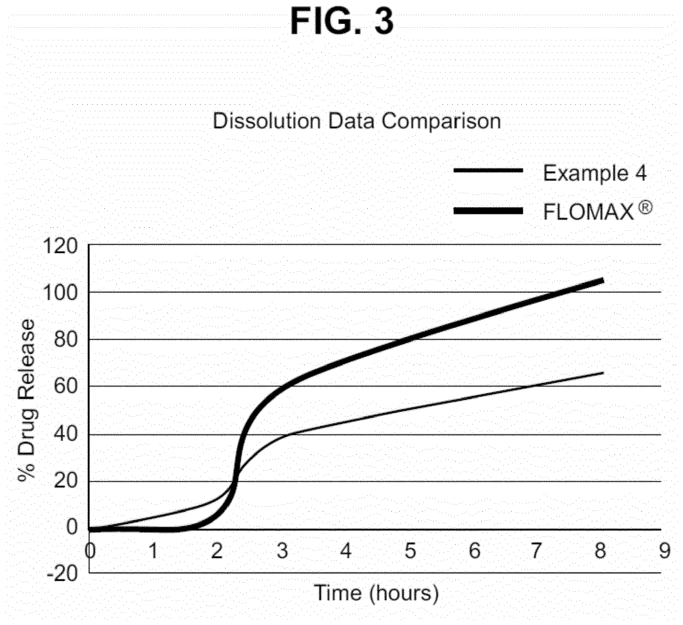

Results: Dissolution of the formulation of Example 4 was compared to FLOMAX® capsules. The results are shown in Fig. 3. The two curves were sufficiently similar to justify further studies *in vitro* and in vivo.

### Example 8: (Actual): Clinical Studies

Objective: The primary objective of this study was to establish the bioequivalence of the formulation of Example 4 ("Tamsulosin DRS" hereinbelow) to FLOMAX® capsules under both fasted and fed conditions. Secondary objectives included assessing the effect of food, or the lack thereof, on the pharmacokinetics (PK) of Tamsulosin DRS; and assessing the safety and tolerability of a single dose of Tamsulosin DRS with and without food.

The study was designed as a two-stage study. Potential subjects were screened for study eligibility within 30 days before the start of the study on Day 0/Period 1.

Stage 1: Formulation Assessment and Dosing Scheme: Stage 1 was an open-label, partially randomized, three-period crossover design. Subjects were randomized into one of two sequences. A total of 12 subjects under fasted and fed conditions were enrolled in Stage 1. Dropouts were not replaced. The dosing scheme was as follows:

| | Period 1 | Period 2 | Period 3 |
|---|---|---|---|
| Dosing Day | Day 0 | Day 7 | Day 14 |
| Formulation 1A (n = 6) | FLOMAX® (Fed) | Tamsulosin DRS (Fasting) | Tamsulosin DRS (Fed) |
| Formulation 1B (n = 6) | FLOMAX® (Fed) | Tamsulosin DRS (Fed) | Tamsulosin DRS (Fasting) |

After Stage 1, bioanalytical analysis of the samples was conducted.

Stage 2: Bioequivalence and Food Effect: Stage 2 was an open-label, randomized, single-center, single-dose, 4-period, crossover, bioavailability study. In each period, the subject received a single dose of either Tamsulosin DRS (test) or FLOMAX® (reference). A total of 36 subjects were enrolled in Stage 2. Dropouts were not replaced unless subject enrollment fell below 30 completed subjects. The dosing scheme was as follows:

| | Period 4 | Period 5 | Period 6 | Period 7 |
|---|---|---|---|---|
| Dosing Day | Day 42 | Day 49 | Day 56 | Day 63 |
| Group 1 (n = 9) | Tamsulosin DRS (Fasting) | FLOMAX® (Fasting) | Tamsulosin DRS (Fed) | FLOMAX® (Fed) |
| Group 2 (n = 9) | FLOMAX® (Fed) | Tamsulosin DRS (Fasting) | FLOMAX® (Fasting) | Tamsulosin DRS (Fed) |
| Group 3 (n = 9) | Tamsulosin DRS (Fed) | FLOMAX® (Fed) | Tamsulosin DRS (Fasting) | FLOMAX® (Fasting) |
| Group 4 (n = 9) | FLOMAX® (Fasting) | Tamsulosin DRS (Fed) | FLOMAX® (Fed) | Tamsulosin DRS (Fasting) |

Inclusion Criteria: Subjects had to fulfill all of the following inclusion criteria to be eligible for participation in the study unless otherwise specified: 1. Healthy adult male volunteers 18 and 60 years of age (inclusive). 2. Able to understand and provide signed informed consent. 3. Willing to comply with the requirements of the study. 4. Seated blood pressure between 100 and 140 mmHg systolic and between 60 and 90 mmHg diastolic (inclusive). 5. Seated pulse rate between 45 and 100 beats/minute (inclusive). 6. Normally active and otherwise judged to be in good health on the basis of medical history and physical examination. 7. Body mass index (BMI) 19.0 and 32.0 kg/m2. 8. Body weight > 55 kg.

Exclusion Criteria: Subjects were not enrolled in the study if they met any of the following exclusion criteria: 1. History of any clinically significant cardiovascular, hepatic, renal, pulmonary, hematologic, gastrointestinal, endocrine, immunologic, dermatologic, or neurologic disease. 2. Any use of prescription medications within 28 days prior to check-in for Period 1 for Stage 1 and Period 4 for Stage 2. Currently taking any medication for treatment of hypertension. Currently taking any medication for treatment of hypercholesterolemia. 3. Any use of over-the-counter (OTC) medicines within 7 days prior to check-in for Period 1 for Stage 1 and Period 4 for Stage 2. 4. History of allergic reaction to Tamsulosin or other related drugs. 5. Abnormal and clinically relevant (as determined by the principal investigator [PI]) electrocardiogram (ECG) tracing. 6. Clinically significant illness or surgery within 28 days prior to dosing (including flu, flu-like symptoms, diarrhea, vomiting) or acute illness at the time of either the pre-study medical evaluation or dosing. 7. Use of any medication known to alter hepatic enzyme activity within 28 days prior to the initial dose of study medication (eg, omeprazole or other proton pump inhibitors [PPIs]). 8. Positive test for hepatitis B, hepatitis C, or human immunodeficiency virus (HIV) at screening. 9. Any clinically significant abnormal laboratory test results found during medical screening as determined by the Investigator. The definition of clinically significant will be related to the normal levels of each test at the local laboratory conducting the test. NOTE: A test value above or below the normal range does not necessarily indicate that the value is "clinically significant." The determination should be made at the discretion of the Investigator with consultation, when necessary, with the Medical Monitor. 10. Alanine aminotransferase (ALT) or aspartate aminotransferase (AST) levels > 1.25 times the upper limit of normal at screening or check-in for Period 1. 11. A positive drug screen or continine screen. 12. History of use of any nicotine products in the 3 months preceding the study start (first dose). 13. A history of major mental illness that in the opinion of the Investigator may affect the ability of the subject to participate in the study. Institutionalized subjects will not be eligible for participation. 14. Exposure to any investigational agent within 30 days prior to study start (first dose). 15. Exposure to Tamsulosin within 30 days prior to study start (first dose). 16. Subject has made a donation (standard donation amount or more) of blood or blood products (with the exception of plasma as noted below) within 56 days prior to the study start (first dose). 17. Subject has made a plasma donation within 7 days prior to the study start (first dose). 18. Subject has a condition the Investigator believes would interfere with the ability to provide informed consent or comply with study instructions, or that might confound the interpretation of the study results or put the subject at undue risk.

Study Procedures: Screening: Potential subjects will be screened for study eligibility within 30 days before the start of the study on Day 0/Period 1 for Stage 1 and within 30 days of Day 42/Period 4 for Stage 2. Subjects will be instructed to fast for at least 8 hours (for laboratory profiles) before arriving for the screening visit. Screening evaluations will include the following: 6. Signed informed consent; 7. Determination of eligibility, inclusion and exclusion criteria (as many as can be determined at the screening visit); 8. Medical history; 9. Previous (used in the previous 30 days) and concomitant medication (currently using); 10. Physical examination, including height and weight without shoes; 11. BMI determination; 12. Vital signs (blood pressure, pulse, respirations, and temperature) after sitting for at least 3 minutes; 13. 12-lead ECG, supine for at least 3 minutes; 14. Laboratory profiles including hematology, chemistry panels, and urinalysis; 15. HIV antibody, hepatitis B surface antigen, and antibody titer against hepatitis C; 16. Toxicology screen for drugs of abuse and alcohol breathalyzer test. Results must be negative for subjects to continue in the study; 17. Subjects will be instructed in the following restrictions: a. Stop the use of prescription medications within 28 days prior to check-in for Period 1 (Stage 1) or Period 4 (Stage 2). b. Stop the use of OTC medicines within 7 days prior to check-in for Period 1 (Stage 1) or Period 4 (Stage 2) (including any non-steroidal anti-inflammatory drugs [NSAIDs] and aspirin-containing medications). c. Stop the use of vitamins or herbal supplements within 7 days prior to check-in for Period 1 (Stage 1) or Period 4 (Stage 2). d. Abstain from foods containing poppy seeds within 24 hours prior to check-in for each study period. e. Abstain from consumption of alcohol-containing products from 48 hours prior to check-in until after the last sample collection of each study period. f. Abstain from foods or beverages containing caffeine, xanthine derivatives or xanthine-related compounds, or energy drinks from 24 hours prior to check-in for each study period. g. Abstain from foods or beverages containing grapefruit and/or Seville oranges from 7 days prior to check-in for Period 1 (Stage 1) or Period 4 (Stage 2) and throughout the study. h. Abstain from St. Jon's wort from screening and throughout the study. i. Abstain from use of tobacco or other nicotine-containing products from screening throughout the study. j. Abstain from use of androgens or anabolic steroids from screening throughout the study.

Check-in (for each period): Subjects will enter the facility for check-in at approximately 4:00 PM on the day before dosing days (check-in on Day -1 for dosing on Day 0, check-in on Day 6 for dosing on Day 7, check-in on Day 13 for dosing on Day 14, check-in on Day 41 for dosing on Day 42, check-in on Day 48 for dosing on Day 49, check-in on Day 55 for dosing on Day 56, and check-in on Day 62 for dosing on Day 63). 1. The inclusion/exclusion criteria will be reviewed and continuing eligibility will be reviewed and assessed. 2. A baseline 12-lead ECG will be performed on Day -1 (Stage 1) and Day 41 (Stage 2). 3. A drug screen and alcohol breathalyzer test will be performed at check-in for each study period. Results must be negative to continue in the study. 4. Any changes in medical history of the subject since the screening visit will be documented and added to the medical history. Clinically significant changes in medical history since Period 1 (Stage 1) or Period 4 (Stage 2) dosing will be recorded as adverse events (AEs). 5. Clinical chemistries and hematology will be collected and assessed for any clinically significant abnormal values. 6. Subjects will be asked about any medications taken since the screening visit or previous study period and adherence to restrictions. 7. A standardized evening meal will be served to the subjects between 5:00 to 7:00 pm or at the normal time of the clinic. An optional snack will be provided at least 10.5 to 11 hours prior to the scheduled dosing (the following day). A supervised fast of at least 10 hours will start after this snack, except for the protocol-defined high-fat meal. 8. The Investigator will review all of the above information and lab results and determine continuing eligibility of the subject prior to each study drug administration.

Study Period 1 (Stage 1) or Period 4 (Stage 2): 1. Assess the subject's continued qualification for the study based on the inclusion/exclusion criteria and compliance with the requirements for fasting and prescription and nonprescription drugs, herbal medications, and other protocol-outlined food restrictions. 2. Prior to the administration of study drug in Period 1 (Stage 1) or Period 4 (Stage 2), subjects will be randomized to a treatment sequence.

In Each Study Period: 3. On the morning of Day 0, Day 7, Day 14, Day 42, Day 49, Day 56 and Day 63, subjects will receive Tamsulosin DRS or Flomax (fasting or fed), after a supervised overnight fast of at least 10 hours. Food will be controlled and standardized for each housing period and for all subjects. Following an overnight fast of at least 10 hours, randomized subjects will receive a standardized high-fat, high-calorie meal 30 minutes before drug administration. An example meal would be two eggs fried in butter, two strips of bacon, two slices of toast with butter, four ounces of hash brown potatoes and eight ounces of whole milk. Substitutions in this test meal can be made as long as the meal provides a similar amount of calories from protein, carbohydrates, and fat and has comparable meal consistency. Subjects must eat the total contents of this meal in 30 minutes or less. At approximately 4.5 and 9.5 hours post dosing of each study period standardized meals will be provided. An evening snack is allowed at approximately 14 hours post dosing of each study period. 4. Prior to the administration of study drug (within 120 minutes prior to dosing), subjects will have vital signs (blood pressure, pulse, temperature, and respiration) performed after sitting for at least 3 minutes prior to the assessment. 5. Blood samples for PK analysis will be taken at the following time points:

| | Analytes | | | Analytes | | | Analytes |
|---|---|---|---|---|---|---|---|
| Time (hours) | Tamsulosin | | Time (hours) | Tamsulosin | | Time (hours) | Tamsulosin |
| t01 | X | | 5 | X | | 18 | X |
| 1 | X | | 6 | X | | 24 | X |
| 2 | X | | 8 | X | | 30 | X |
| 3 | X | | 10 | X | | 36 | X |
| 4 | X | | 12 | X | | 48 | X |

'The to sample will be drawn within 120 minutes prior to dosing on the dosing day. Based on a reported T ½ of 5 hrs.

6. Blood samples for assessment of clinical chemistry and hematology will be collected at screening, at each study check-in and study exit. a. Clinical chemistry (blood chemistry) will consist of ALT, albumin, alkaline phosphatase, AST, blood urea nitrogen (BUN), calcium, carbon dioxide (bicarbonate), chloride, creatinine, glucose, inorganic phosphate, magnesium, potassium, sodium, total bilirubin, total protein, uric acid. b. Hematology will consist of hematocrit (HCT), hemoglobin (HGB), white blood cell (WBC) count, and platelet count and differential. 7. Urine samples for urinalysis will be collected at screening and study exit. a. Urinalysis will consist of glucose, bilirubin, specific gravity, blood, pH, protein, urobilirubin, nitrite, leucocyte esterase and microscopic assessment (if deemed necessary through evidence of cellular abnormality). 8. All subjects will fast no less than 4 hours after their scheduled dosing time on each dose day. After the 4-hour fast post dose on each dosing day, standard meals will be provided at specified times during the confinement period. Meal plans will be identical for each study period. 9. Monitoring for treatment-emergent adverse events (TEAEs) will begin as soon as the subject receives the first dose of study drug and continue until 48 hours after the last dose or early termination. 10. Vital signs: Seated blood pressure and pulse rate will be measured at approximately 12 hours and 24 hours post-dose [± 15 minutes] on Day 0, Day 7, Day 14, Day 42, Day 49, Day 56 and Day 63 (after sitting for at least 3 minutes prior to the assessment), and at any other time deemed medically necessary.

Release from Clinic or Early Termination: Subjects will be confined in the study center until at least 48 hours after dosing in each period. Subjects will be discharged after all protocol-defined procedures are completed for that period. A reminder about the washout period (food/medication restrictions) and when to return for the next study period (washout of a minimum of 7 days between doses) will be provided. The following evaluations will be performed after the final blood draw (Day 16 Stage 1 and Day 65 Stage 2) or at early withdrawal: 11. Review of AEs and concomitant medications. 12. Physical assessment, including signs of bleeding. 13. Fasting laboratory profile (excluding serology and drug/alcohol test). 14. Physical exam, including signs of bleeding. 15. 12-lead ECG, supine for at least 3 minutes. The subjects will then be discharged from the study center. If a subject withdraws from the study early, every effort will be made to have the subject remain in the clinic until at least 48 hours after dosing. If they cannot remain in the clinic, every effort will be made to have the subject return to the clinic approximately 48 hours after dosing to have the above evaluations performed. Total study duration is approximately 17 days for Stage 1 and 25 days for Stage 2 (not including the screening period). Confinement to the clinical site will be from approximately 4:00 PM of the day before dosing in each study period through 48 hours post-dose (a total of approximately 72 hours for each dosing period).

Criteria for Evaluation: Pharmacokinetics: The following noncompartmental PK parameters will be estimated from the plasma concentration-time course data: AUCt, AUC∞, Cmax, Tmax, kel, Cl/F, t½, and Vd/F. No value for AUC∞, kel, Cl/F, and t½, will be reported for cases that do not exhibit a terminal log-linear phase in the concentration-time data. Single-dose PK parameters will be estimated.

Safety: Safety assessments will include screening, pre-dose and post-dose vital signs, ECGs, clinical laboratory testing (hematology, blood chemistry, and urinalysis), documentation of AEs, and physical examinations.

Statistical Methods: Pharmacokinetics: Pharmacokinetic parameters (AUCt, AUC∞, Cmax, Tmax, kel, Cl/F, t½, and Vd/F will be estimated following noncompartmental analysis of plasma concentration time-course data for Tamsulosin on the days subjects received Tamsulosin. Arithmetic means, standard deviations, and coefficients of variation will be calculated for all PK parameters. Additionally, geometric means will be calculated for AUCt, AUC∞, and Cmax. Analyses of variance (ANOVA) will be performed on the In-transformed PK parameters AUCt, AUC∞, and Cmax. Each ANOVA will include calculation of least-squares means (LSMs), the differences between adjusted means, and the standard error associated with these differences. Ratios of means will be calculated using the LSM for In-transformed AUCt, AUC∞, and Cmax. The 90% confidence interval [CI] for the difference in each analyte will be calculated for the parameters AUCt, AUC∞, and Cmax using In-transformed concentration data. The CIs will be expressed as a percentage relative to the LSM. The following comparisons will be made:

**Stage 1:**

| Comparison | Test | Reference |
|---|---|---|
| #1 | Tamsulosin DRS (Fed) | FLOMAX® (Fed) |
| #2 | Tamsulosin DRS (Fasting) | FLOMAX® (Fed) |
| #3 | Tamsulosin DRS (Fasting) | Tamsulosin DRS (Fed) |

**Stage 2:**

| Comparison | Test | Reference |
|---|---|---|
| #1 | Tamsulosin DRS (Fasting) | FLOMAX® (Fasting) |
| #2 | Tamsulosin DRS (Fed) | FLOMAX® (Fed) |
| #3 | Tamsulosin DRS (Fed) | Tamsulosin DRS (Fasting) |

If the 90% Cis for the ratios of geometric least-squares means derived from the analyses of the In-transformed PK parameters, AUCt, AUC∞, and Cmax for comparison #9, outlined above, are within the acceptable range, then no food interaction with the Tamsulosin DRS will be concluded.

### Results:

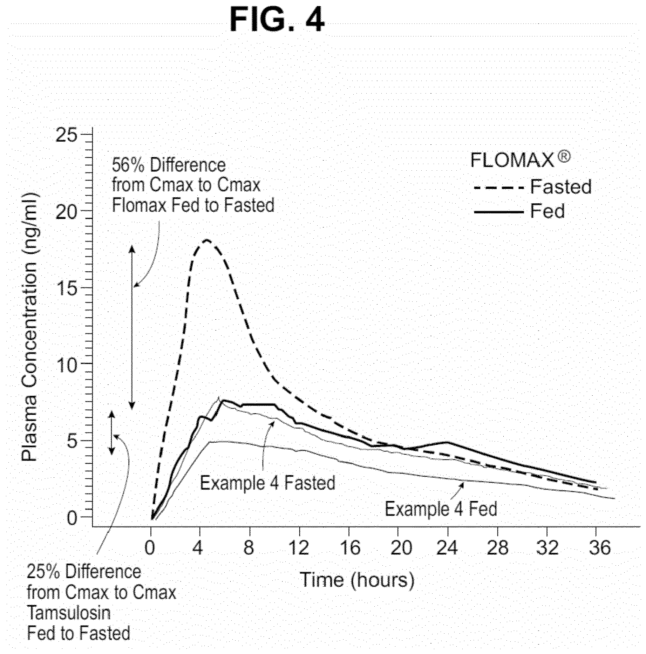

Tamsulosin DRS showed the surprising result that the Tamsulosin DRS under fasted conditions provided a similar PK profile when compared with FLOMAX® fed. The results are shown in Fig. 4. The FLOMAX® package insert dose and administration instructions state "It should be administered approximately one-half hour following the same meal each day". These instructions are due to the much higher systemic exposure to FLOMAX® when dosed in the fasted state. The study data demonstrated a 56% difference in the Cmax for the FLOMAX® fed versus fasted. Tamsulosin DRS in the same study demonstrated only a 25% difference in the Cmax for the fed versus the fasted state. Overall the Tamsulosin DRS fasted PK curve was similar to the FLOMAX® fed curve.

Although what has been described hereinabove relates to Tamsulosin particularly, persons skilled in the art will appreciate that the methods utilized herein can be used in a similar manner with other active ingredients. The inventive methods applied to such other active ingredients and the resulting dosage forms are considered to be part of the present invention. A list of suitable other actives can be selected from the list Oral Dosage Forms That Should Not Be Crushed 2015 or the list Oral Dosage Forms That Should Not Be Crushed 2016, both of which can be found on the website ismp.org. These lists are incorporated herein by reference. Other active ingredients will be present in the resulting dosage forms in amounts effective for the treatment purposes for which the other active ingredients are utilized. These other active ingredients will be combined with the inventive matrix materials and processed as described herein to yield sachets and the other dosage forms described herein to provide relief against dysphagia and possibly also food effects. A specific contemplated active ingredient from such list is solifenacin or a pharmaceutically acceptable salt thereof, especially solifenacin succinate, the active ingredient in VESIcare®. Contemplated embodiments further include any one of the foregoing embodiments related to tamsulosin or a pharmaceutically acceptable salt thereof, wherein the tamsulosin or tamsulosin salt is substituted for by solifenacin or a pharmaceutically acceptable salt thereof in an effective amount, for example 1-15 mg, especially 5 or 10 mg, of the solifenacin or the pharmaceutically acceptable salt thereof for the treatment of overactive bladder, incontinence, and frequent urination.

The Tamsulosin can also be combined in the embodiments described herein with other active ingredients having a complementing effect against BPH, for example, 5α-reductase inhibitors, especially AVODART® (dutasteride = (1S,3aS,3bS,5aR,9aR,9bS,11aS)-N-[2,5-bis(trifluoromethyl)phenyl]-9a,11a-dimethyl-7-oxo-1,2,3, 3a,3b,4,5,5a,6,9b,10,11-dodecahydroindeno[5,4-f]quinoline-1-carboxamide) or a pharmaceutically acceptable salt thereof. Such a combination may have usefulness against androgenetic alopecia. The dosage of dutasteride in such a combination would be 0.5 mg, or another amount effective to treat BPH.

## Claims

1. A pharmaceutical dosage form comprising a plurality of particles providing controlled release of Tamsulosin or a physiologically acceptable salt thereof;
wherein each of said particles comprises a controlled release matrix in which the Tamsulosin or the physiologically acceptable salt thereof is embedded;
wherein said controlled release matrix comprises a mixture of a first cellulose derivative and a second cellulose derivative; wherein said first cellulose derivative differs from said second cellulose derivative; wherein said first cellulose derivative and said second cellulose derivative each are independently of one another selected from the group consisting of cellulose ethers and cellulose esters; and wherein the total content of said first cellulose derivative and said second cellulose derivative is at least 50 wt.-%, relative to the total weight of the plurality of particles; and
wherein the particles are not coated with an enteric coating material.

2. The pharmaceutical dosage form according to claim 1, wherein the particles have an average particle size within the range of from 160 µm to 415 µm, preferably from 175 µm to 400 µm.

3. The pharmaceutical dosage form according to claim 1 or 2, wherein
- a fraction of said plurality of particles having a particle size below 160 µm amounts to not more than 95.0 wt.-%, preferably not more than 90 wt.-% of the total weight of said plurality of particles; or
- a fraction of said plurality of particles having a particle size above 415 µm amounts to not more than 95.0 wt.-%, preferably not more than 90 wt.-% of the total weight of said plurality of particles.

4. The pharmaceutical dosage form according to any of the preceding claims, wherein the total content of said first cellulose derivative and said second cellulose derivative is at least 55 wt.-%, more preferably at least 60 wt.-%, still more preferably at least 65 wt.-%, yet more preferably at least 70 wt.-%, even more preferably at least 75 wt.-%, most preferably at least 80 wt.-%, and in particular at least 85 wt.-%, relative to the total weight of the plurality of particles.

5. The pharmaceutical dosage form according to any of the preceding claims, wherein
- the content of said first cellulose derivative is at least 10 wt.-%, more preferably at least 15 wt.-%, still more preferably at least 20 wt.-%, yet more preferably at least 25 wt.-%, even more preferably at least 30 wt.-%, most preferably at least 35 wt.-%, and in particular at least 40 wt.-%, relative to the total weight of the plurality of particles; and/or
- the content of said second cellulose derivative is at least 10 wt.-%, more preferably at least 15 wt.-%, still more preferably at least 20 wt.-%, yet more preferably at least 25 wt.-%, even more preferably at least 30 wt.-%, most preferably at least 35 wt.-%, and in particular at least 40 wt.-%, relative to the total weight of the plurality of particles; and/or
- the content of Tamsulosin or the physiologically acceptable salt thereof is at least 3.0 wt.-%, more preferably at least 4.0 wt.-%, still more preferably at least 5.0 wt.-%, yet more preferably at least 6.0 wt.-%, even more preferably at least 7.0 wt.-%, most preferably at least 8.0 wt.-%, and in particular at least 9.0 wt.-%, relative to the total weight of the plurality of particles.

6. The pharmaceutical dosage form according to any of the preceding claims, wherein
- the content of said first cellulose derivative is within the range of 45±40 wt.-%, preferably 45±35 wt.-%, more preferably 45±30 wt.-%, still more preferably 45±25 wt.-%, yet more preferably 45±20 wt.-%, even more preferably 45±15 wt.-%, most preferably 45±10 wt.-%, and in particular 45±5 wt.-%, relative to the total weight of the plurality of particles; and/or
- the content of said second cellulose derivative is within the range of 45±40 wt.-%, preferably 45±35 wt.-%, more preferably 45±30 wt.-%, still more preferably 45±25 wt.-%, yet more preferably 45±20 wt.-%, even more preferably 45±15 wt.-%, most preferably 45±10 wt.-%, and in particular 45±5 wt.-%, relative to the total weight of the plurality of particles; and/or
- the content of the Tamsulosin or the physiologically acceptable salt thereof is within the range of 10±8 wt.-%, preferably 10±7 wt.-%, more preferably 10±6 wt.-%, still more preferably 10±5 wt.-%, yet more preferably 10±4 wt.-%, even more preferably 10±3 wt.-%, most preferably 10±2 wt.-%, and in particular 10±1 wt.-%, relative to the total weight of the plurality of particles, relative to the total weight of the plurality of particles.

7. The pharmaceutical dosage form according to any of the preceding claims, wherein the relative weight ratio of said first cellulose derivative to said second cellulose derivative is within the range of from 5:1 to 1:5; preferably from 4.5:1 to 1:4.5; more preferably from 4:1 to 1:4, still more preferably from 3.5:1 to 1:3.5, yet more preferably from 3:1 to 1:3, even more preferably from 2.5:1 to 1:2.5, most preferably from 2:1 to 1:2, and in particular from 1.5:1 to 1:1.5.

8. The pharmaceutical dosage form according to any of the preceding claims, wherein said first cellulose derivative and said second cellulose derivative each are independently of one another selected from the group consisting of ethyl cellulose, cellulose acetate phthalate, hydroxypropyl methyl cellulose, hydroxypropyl methyl cellulose acetate succinate, and hydroxypropyl methyl cellulose phthalate.

9. The pharmaceutical dosage form according to any of the preceding claims, wherein
- the first cellulose derivative is ethyl cellulose; and/or
- the second cellulose derivative is selected from the group consisting of hydroxypropyl methyl cellulose, hydroxypropyl methyl cellulose acetate succinate, and hydroxypropyl methyl cellulose phthalate.

10. The pharmaceutical dosage form according to any of the preceding claims, wherein said plurality of particles
- are free flowing, non-compacted particles; and/or
- have a particle size in the range of from 100 µm to 600 µm, preferably 150 µm to 450 µm.

11. The pharmaceutical dosage form according to any of the preceding claims, wherein said plurality of particles
- are not coated with any coating material; and/or
- do not contain one or more materials selected from the group consisting of microcrystalline cellulose, polyethylene oxide, alginic acid, alginate salts, lactose, and glycerol behenate; preferably do not contain any of the foregoing materials; and/or
- essentially consists of, preferably consist of said first cellulose derivative, said second cellulose derivative and Tamsulosin or the physiologically acceptable salt thereof.

12. The pharmaceutical dosage form according to any of the preceding claims, for use in the treatment of benign prostatic hyperplasia.

13. The pharmaceutical dosage form for use according to claim 12, wherein the pharmaceutical dosage form is administered orally once daily.

14. A sachet comprising a pharmaceutical dosage form according to any of the preceding claims.

15. The sachet according to claim 13, wherein the sachet
- comprises the pharmaceutical dosage form and a gas selected from air, nitrogen, oxygen, argon, carbon dioxide and any mixture of the foregoing; and/or
- is made from a material selected from the group consisting of polymers, metal, paper, and any combination thereof; and/or
- is transparent or comprises a transparent portion; and/or
- is made from a material having a thickness of not more than 1000 µm, more preferably not more than 900 µm, still more preferably not more than 800 µm, yet more preferably not more than 700 µm, even more preferably not more than 600 µm, most preferably not more than 500 µm, and in particular not more than 400 µm; and/or
- has an inner volume of not more than 1000 cm³, more preferably not more than 500 cm³, still more preferably not more than 250 cm³, yet more preferably not more than 100 cm³, even more preferably not more than cm³ µm, most preferably not more than 75 cm³, and in particular not more than 50 cm³.
